(19) Europäisches Patentamt / European Patent Office / Office européen des brevets

(11) **EP 4 375 647 A1**

(12) **EUROPEAN PATENT APPLICATION**
published in accordance with Art. 153(4) EPC

(43) Date of publication:
**29.05.2024 Bulletin 2024/22**

(21) Application number: **22845776.8**

(22) Date of filing: **04.07.2022**

(51) International Patent Classification (IPC):
**G01N 21/78** $^{(2006.01)}$    **G01N 21/64** $^{(2006.01)}$
**G01N 33/543** $^{(2006.01)}$

(52) Cooperative Patent Classification (CPC):
**G01N 21/64; G01N 21/78; G01N 33/543**

(86) International application number:
**PCT/JP2022/026644**

(87) International publication number:
**WO 2023/002842 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.07.2021 JP 2021120870**

(71) Applicant: **FUJIFILM Corporation**
**Tokyo 106-8620 (JP)**

(72) Inventor: **ISHII, Hiroyasu**
**Ashigarakami-gun, Kanagawa 258-8538 (JP)**

(74) Representative: **Hughes, Andrea Michelle**
**Dehns Germany**
**Theresienstraße 6-8**
**80333 München (DE)**

(54) **IMMUNOCHROMATOGRAPH INSPECTION APPARATUS**

(57)    The immunochromatographic assay apparatus includes a loading part in which a cartridge including an assay strip having an assay region of which a density varies depending on an amount of a test substance included in a sample is attachably and detachably loaded, a detection unit that detects the density of the assay region, a processor configured to perform a positive-negative determination of whether the sample is positive or negative based on the density acquired from the detection unit. The processor is configured to start acquisition of the density from the detection unit from a preset time point before the density of the assay region is saturated, and perform saturation point determination of whether or not the density has reached a saturation point based on a time change of the density, and in a case where it is determined in the saturation point determination that the density has reached the saturation point, perform the positive-negative determination.

FIG. 13

**EP 4 375 647 A1**

**Description**

**BACKGROUND OF THE INVENTION**

1. Field of the Invention

The present disclosure relates to an immunochromatographic assay apparatus.

2. Description of the Related Art

JP2009-139254A describes an immunochromatographic kit for performing an assay of whether a sample is positive or negative, that is, whether or not the sample contains a test substance, using an immunochromatographic method. This immunochromatographic kit is called an assay cartridge or the like. An assay strip to which a sample is supplied and a case in which the assay strip is accommodated are included.

An assay region in which the color development state changes depending on whether the sample is positive or negative is provided on the surface of the assay strip. An antibody that specifically binds to an antigen, which is a test substance, is immobilized on the assay region. In a case where a sample containing an antigen is developed in the assay region, the antigen binds to the antibody immobilized on the assay region, and is captured in the assay region. In a case where the antigen is modified with a labeling substance, the color development state of the assay region changes depending on the capture amount of the antigen captured in the assay region by the action of the labeling substance. The color development state appears as, for example, a density change, and it is determined whether the sample is positive or negative by detecting the shade of the density of the assay region based on a preset threshold value. The labeling substance includes a labeling substance of which the density is amplified by an amplifying liquid and a labeling substance that emits fluorescence by irradiation with excitation light.

The cartridge described in JP2012-103150A is a type that includes an amplifying liquid pod for accommodating an amplifying liquid and that amplifies the density of the assay region by the amplifying liquid.

JP2012-103150A discloses an analysis apparatus (corresponding to an assay apparatus) that includes a loading part in which an assay cartridge is loaded and that optically analyzes the density of an assay region. The analysis apparatus includes a sensor that optically detects the density of the assay region and a display unit that displays a determination result to determine whether the sample is positive or negative. The cartridge described in JP2012-103150A is a type that includes an amplifying liquid pod for accommodating an amplifying liquid and that amplifies the density of the assay region by the amplifying liquid.

**SUMMARY OF THE INVENTION**

In the immunochromatographic assay, even in a case where the sample is positive, there are individual differences in the density change rate at which the density of the assay region changes depending on the amount of the test substance included in the sample, and the time until the threshold value is exceeded varies. Therefore, in order to accurately determine whether the sample is positive or negative based on the density of the assay region (hereinafter, positive-negative determination), for example, after starting the supply of the sample or the amplifying liquid, the determination is necessary to be performed after waiting a sufficient time in consideration of the individual difference in the density change rate of the assay region.

However, in a case where the determination timing of the positive-negative determination is temporally managed in consideration of individual differences as described above, it is necessary to set a waiting time until the positive-negative determination in accordance with the time at which the density change rate is the slowest. In that case, even for a sample having a relatively fast density change rate, a relatively long waiting time becomes rate-controlling, and thus in a case where an assay is performed with respect to a plurality of assay cartridges, there is a problem that the throughput of the assay decreases as a whole.

The present disclosure has been made in view of the above circumstances, and an object of the present disclosure is to provide an immunochromatographic assay apparatus capable of improving the throughput of the assay as compared with the prior art.

The immunochromatographic assay apparatus according to a first aspect of the present disclosure comprises

a loading part in which a cartridge including an assay strip having an assay region of which a density varies depending on an amount of a test substance included in a sample is attachably and detachably loaded, a detection unit that detects the density of the assay region, and a processor configured to perform a positive-negative determination of whether the sample is positive or negative based on the density acquired from the detection unit, in which the processor is configured to

start acquisition of the density from the detection unit from a preset time point before the density is saturated, and perform saturation point determination of whether or not the density has reached a saturation point based on a time change of the density, and in a case where it is determined in the saturation point determination that the density has reached the saturation point, perform the positive-negative determination.

In the immunochromatographic assay apparatus according to the first aspect of the present disclosure, the processor may be configured to, in the saturation point determination, derive an increase rate of the density from a time change of the density, and in a case where the increase rate of the density decreases from a peak value to a predetermined value or less, determine that the density has reached the saturation point.

The immunochromatographic assay apparatus according to a second aspect of the present disclosure comprises a loading part in which a cartridge including an assay strip having an assay region of which a density varies depending on an amount of a test substance included in a sample is attachably and detachably loaded,

a detection unit that detects the density of the assay region, and
a processor configured to perform a positive-negative determination of whether the sample is positive or negative based on the density acquired from the detection unit,
in which the processor is configured to

start acquisition of the density from the detection unit from a preset time point before the density is saturated, and
perform the positive-negative determination based on the density before saturation.

In the immunochromatographic assay apparatus according to the second form of the present disclosure, the processor may be configured to, in the positive-negative determination, compare the density before the saturation with a predetermined first density threshold value, and in a case where a comparison result satisfies a predetermined first condition, determine that the sample is positive.

The first condition may be a case where the density before the saturation exceeds the first density threshold value.

In a case where the comparison is performed a plurality of times, the first condition may be a case where the number of times that the density before the saturation exceeds the first density threshold value reaches a predetermined number of times, or a case where an average value of the densities before the saturation continuously acquired a plurality of times exceeds the first density threshold value.

In the immunochromatographic assay apparatus according to the second aspect of the present disclosure, the processor may be configured to, based on actually measured data indicating a time change of the density before the saturation, which is obtained by acquiring from the detection unit a plurality of times, and reference data, which is prepared in advance, indicating a time change of a reference density of a reference sample that includes a known amount of the test substance, derive a predicted saturation point of the density, which predicted from the actually measured data, compare the predicted saturation point with a predetermined second density threshold value, and perform the positive-negative determination.

The second density threshold value is preferably set to be higher than a threshold value used in a case of performing the positive-negative determination based on the density of a saturation point of the actually measured data.

In the immunochromatographic assay apparatus according to the second aspect of the present disclosure, the reference data includes a plurality of reference data for a plurality of reference samples including the test substances having known amounts different from each other, and the processor may be configured to derive the predicted saturation point using at least one reference data most approximated to the actually measured data among the plurality of reference data.

In the immunochromatographic assay apparatus according to the second aspect of the present disclosure, the processor may be configured to obtain approximated data that approximates to the actually measured data by interpolation or extrapolation based on one or two of the reference data that relatively approximates to at least the actually measured data among the reference data, and derive the predicted saturation point based on the approximated data.

In the immunochromatographic assay apparatus according to the second aspect of the present disclosure, the reference data is preferably a look-up table or a function showing correlation between time and the density.

In the immunochromatographic assay apparatus according to the second aspect of the present disclosure, the processor may be configured to, in a case where a difference between the predicted saturation point and the second density threshold value is within a predetermined range, perform the positive-negative determination after waiting until the density acquired from the detection unit reaches a saturation point without performing the positive-negative determination based on the predicted saturation point.

In the immunochromatographic assay apparatus according to the first aspect and the second aspect of the present disclosure, the processor may be configured to determine whether or not a predetermined second condition is satisfied, and in a case where the second condition is not satisfied, stop the positive-negative determination, output a determination result with a warning, or perform the positive-negative determination after waiting until the second condition is satisfied.

In the immunochromatographic assay apparatus according to the first aspect and the second aspect of the present disclosure, the second condition may include a condition that a state which affects a determination accuracy of the positive-negative determination does not occur.

In the immunochromatographic assay apparatus according to the first aspect and the second aspect of the present disclosure, in a case where the density of the assay region is amplified by developing an amplifying liquid in the assay region, the second condition may include a condition that air bubbles in the amplifying liquid in the assay region have disappeared.

In the immunochromatographic assay apparatus according to the first aspect and the second aspect of the present disclosure, in a case where the assay strip has a control region in which a color development state changes depending on whether or not at least one of the sample or an amplifying liquid that amplifies the density of the assay region is developed in the assay region, the second condition may include a condition that a color development state of the control region has changed.

In the immunochromatographic assay apparatus according to the first aspect and the second aspect of the present disclosure, a density of the control region changes by the development of the sample or the amplifying liquid, and reference data, which is prepared for determining the density of the control region, indicating a standard time change of density in a case where the sample or the amplifying liquid is developed is defined as second reference data, the processor may be configured to determine whether or not the second condition is satisfied, using the second reference data.

In the immunochromatographic assay apparatus according to the first aspect and the second aspect of the present disclosure, the second condition includes a condition that the color development state of the control region has changed within a preset time, and the processor may be configured to, in a case where the second condition is not satisfied, stop the positive-negative determination and warn an abnormality.

In the immunochromatographic assay apparatus according to the first aspect and the second aspect of the present disclosure, a type that amplifies the density of the assay region by developing an amplifying liquid in the assay region may be used as the cartridge.

According to the immunochromatographic assay apparatus of the present disclosure, it is possible to improve the assay throughput as compared with the prior art.

## BRIEF DESCRIPTION OF THE DRAWINGS

Fig. 1 is a perspective view showing appearance of an immunochromatographic assay apparatus.
Fig. 2 is a perspective view of a cartridge.
Fig. 3 is an exploded perspective view of the cartridge.
Fig. 4A is a partially broken side view showing a state in which a first pressing operation part of the cartridge according to the present disclosure is pushed in, and Fig. 4B is a partially broken side view showing a state in which the first pressing operation part and a second pressing operation part are pushed in.
Fig. 5 is a diagram showing a positional relationship between an assay strip, a multifunctional member, a first amplifying liquid holding part, and a second amplifying liquid holding part, in the cartridge.
Fig. 6 is an explanatory diagram of an immunochromatographic method.
Fig. 7 is a partially broken side view of an assay apparatus in a state where the cartridge is loaded.
Fig. 8 is a schematic diagram showing a general density change of an assay region L1.
Fig. 9 is a schematic diagram showing a general density change of an assay region L1.
Fig. 10 is a schematic diagram showing a time change of a density of an assay region L1 and a time change of an increase rate of the density of the assay region L1.
Fig. 11 is an explanatory diagram of a method of saturation point determination in the assay apparatus according to the first embodiment.
Fig. 12 is a diagram showing a first assay flow.
Fig. 13 is a diagram showing a processing procedure of an assay in the assay apparatus according to the first embodiment.
Fig. 14 is a diagram showing Modification Example 1 of the processing procedure shown in Fig. 13.
Fig. 15 is a diagram showing Modification Example 2 of the processing procedure shown in Fig. 13.
Fig. 16 is a diagram showing a first example of the processing procedure of the assay in the assay apparatus according to the second embodiment.
Fig. 17 is an explanatory diagram of a method of determining a positivity in the assay apparatus according to the second embodiment.
Fig. 18 is explanatory diagrams of a positive-negative determination in the assay apparatus according to the second embodiment, where (A) is a schematic diagram of actually measured data, (B) is a schematic diagram of reference data, and (C) is a schematic diagram showing a method of deriving a predicted saturation point.
Fig. 19 is a diagram showing a second example of the processing procedure of the assay in the assay apparatus according to the second embodiment.
Fig. 20 is a diagram showing a modification example of the second example of the processing procedure of the assay in the assay apparatus according to the second embodiment.

## DESCRIPTION OF THE PREFERRED EMBODIMENTS

Hereinafter, an immunochromatographic assay apparatus (hereinafter, simply referred to as an assay apparatus) according to the embodiment of the present disclosure is described with reference to the drawings.

The constituent elements indicated by the same reference numerals in the respective drawings mean the same constituent elements. However, unless otherwise specified in the specification, each constituent element is not limited to one, and a plurality of each constituent element may be present.

In addition, description of overlapping configurations and reference numerals in the respective drawings may be omitted. In addition, the present disclosure is not limited to the embodiments below, and can be implemented with appropriate modifications, such as omitting a configuration or replacing a configuration with a different configuration within the scope of the object of the present disclosure.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

<First embodiment>

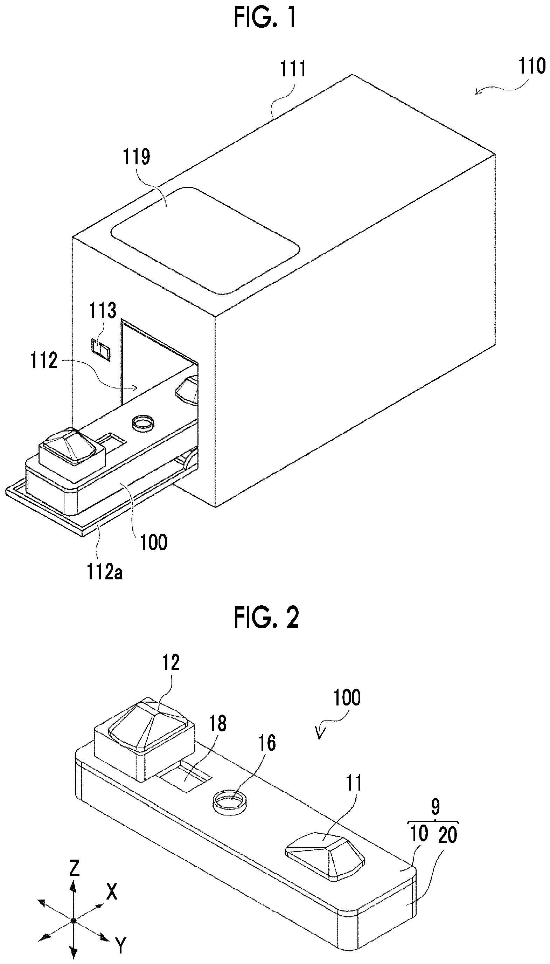

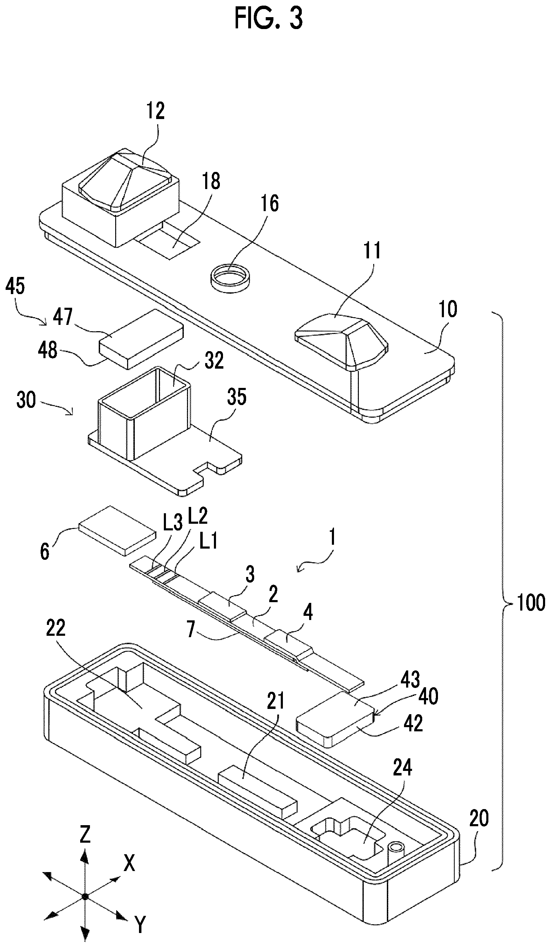

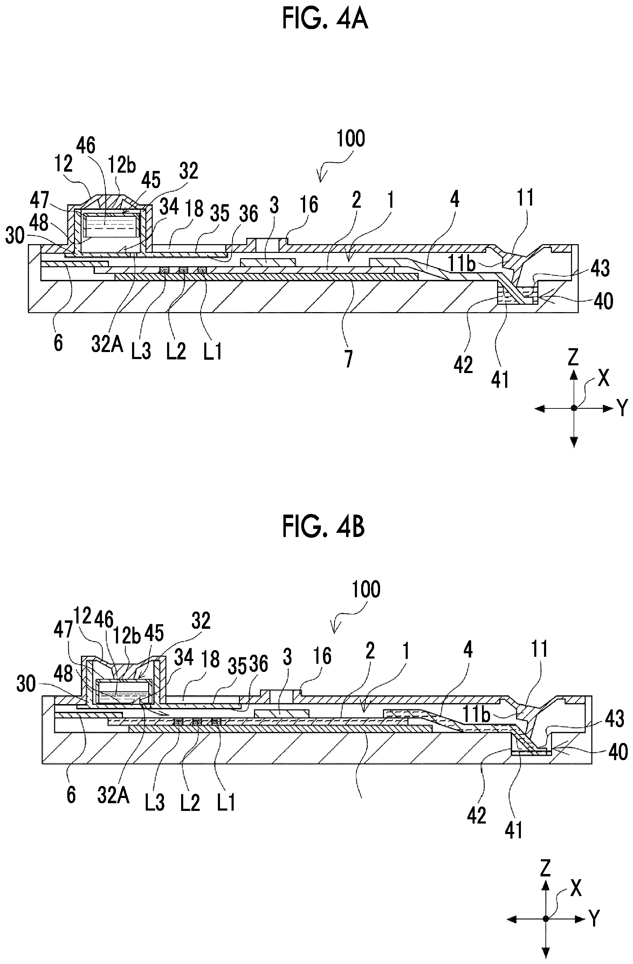

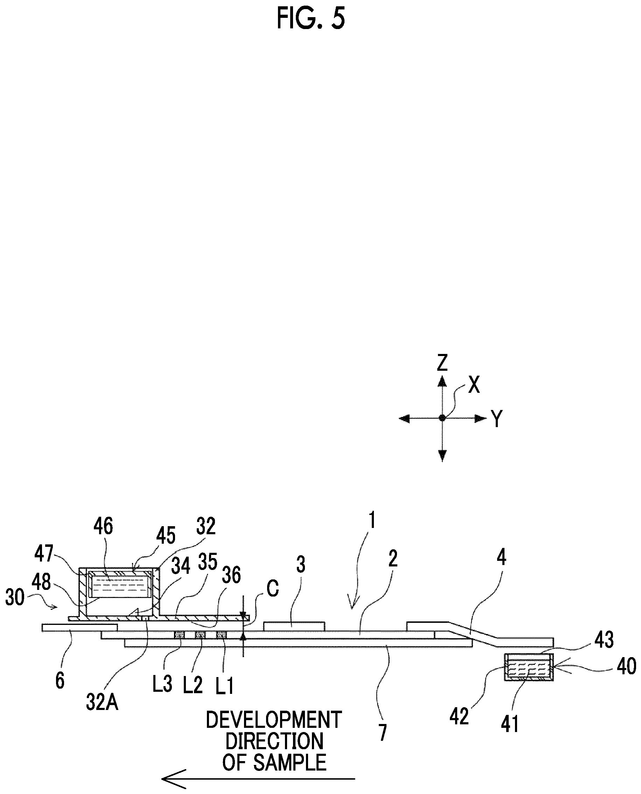

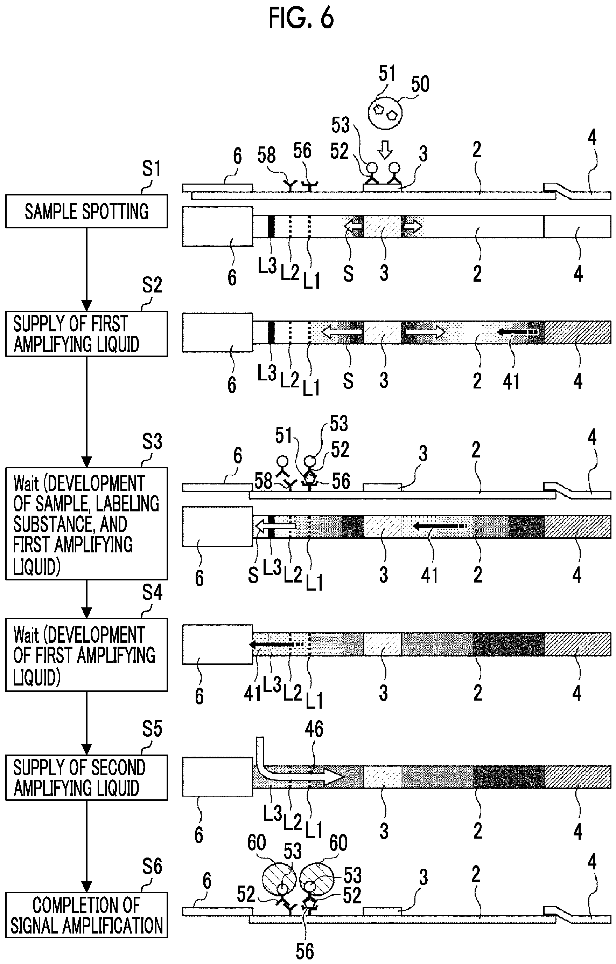

Fig. 1 is a perspective view showing an appearance of an assay apparatus 110 according to a first embodiment. Fig. 2 is an external view of an assay cartridge 100 (Hereinafter, referred to as a cartridge 100) attached to the assay apparatus 110, and Fig. 3 is an exploded perspective view of the cartridge 100. Fig. 4 is a diagram showing a state where a first pressing operation part 11 and a second pressing operation part 12 provided in the cartridge 100 are operated. More specifically, Fig. 4A shows a state in which the first pressing operation part 11 is operated, and Fig. 4B shows a state in which the first pressing operation part 11 and the second pressing operation part 12 are operated. Fig. 5 is a diagram showing the main accommodated components in the cartridge 100. Fig. 6 is an explanatory diagram of an immunochromatographic method.

The cartridge 100 is a single-use type that is used one by one in each sample of assay target. As shown in Fig. 3, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, or a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state where the sample is spotted is loaded. Then, the assay apparatus 110 detects a color development state of the assay region L1 of the loaded cartridge 100, and presents the determination result of whether the sample is positive or negative. In a case where a plurality of samples are assayed, the cartridge 100 for each sample is loaded one by one into the assay apparatus 110.

Hereinafter, the cartridge 100 is described on the premise that the cartridge 100 is loaded into the assay apparatus 110. However, the cartridge 100 of the present example has a configuration that a user can confirm visually whether the sample is positive or negative without using the assay apparatus 110. Such a cartridge 100 is also referred to as an immunochromatographic assay tool, an immunochromatographic assay kit, or the like.

As shown in Fig. 1, the assay apparatus 110 includes a housing 111, and the housing 111 includes a cartridge loading part 112 in which the cartridge 100 is attachably and detachably loaded. As an example, an opening for inserting the cartridge 100 into the housing 111 and an opening and closing lid 112a for opening and closing the opening are provided on the front surface of the housing 111. The opening and closing lid 112a is opened when the cartridge 100 is loaded, the cartridge 100 is inserted into the housing 111, and the opening and closing lid 112a is closed after the cartridge 100 has been loaded into the cartridge loading part 112. The assay is performed in a state where the opening and closing lid 112a is closed.

In addition, a power switch 113 is provided on the front surface of the housing 111, and a monitor 119 is provided on the upper surface of the housing 111. A determination result, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

<Overview of cartridge>

As shown in Fig. 2 and Fig. 3, as an example, the cartridge 100 includes a case 9 constituted of a case main body 20 and a cover member 10. The case 9 is formed of, for example, a resin material. An opening is formed in an upper part of the case main body 20, and in addition to the assay strip 1, a first amplifying liquid holding part 40, a second amplifying liquid holding part 45, and the like are accommodated therein. The cover member 10 covers the opening of the case main body 20 by being attached to the opening part of the case main body 20. The case 9 has an elongated shape as a whole in accordance with the elongated shape of the assay strip 1.

In the present example, a dropping port 16, an observation window 18, a first pressing operation part 11, and a second pressing operation part 12 are provided on an upper part of the case 9 constituted of the cover member 10. Each of these parts is integrally molded with the cover member 10 as an example. The dropping port 16 is an opening for adding dropwise a sample into the inside of the case 9. A boss is vertically provided on the edge of the dropping port 16 toward the upper part.

(Observation window)

An observation window 18 is an opening portion for observing the assay region L1 from the outside, in the present example, the size of the observation window 18 is a size such that, in addition to the assay region L1, the control region L2 and the color development region L3, which is described below, can also be observed. The user can confirm whether the sample is positive or negative by observing the color development state of the assay region L1 in the observation window 18.

(First pressing operation part and second pressing operation part)

As shown in Fig. 3, Fig. 4A, and Fig. 4B, the first pressing operation part 11 is an operating part operated to supply the first amplifying liquid 41 in the first amplifying liquid holding part 40 to the assay strip 1. The second pressing operation part 12 is an operating part operated to supply a second amplifying liquid 46 in the second amplifying liquid holding part 45 to the assay strip 1. As described later, the first amplifying liquid 41 and the second amplifying liquid 46 are amplifying liquids for amplifying the color development in the assay region L1 in a case where the sample is positive.

As shown in Fig. 4A, in a case where a pressing force is applied to the first pressing operation part 11 by a pressing operation by a user, or the like, the first pressing operation part 11 is deformed. As shown in Fig. 3, as an example, the first pressing operation part 11 has a quadrangular pyramid shape, and in a case where a pressing force is applied from above to a region including the apex of the quadrangular pyramid, as shown in Fig. 4A, the first pressing operation part 11 is deformed such that the apex of the quadrangular pyramid sinks into the inside of the case 9. The first pressing operation part 11 of the present example is provided with a protruding part 11b that abuts on the sheet member 43 of the first amplifying liquid holding part 40, which is described below. In a case where the first pressing operation part 11 sinks, the protruding part 11b of the first pressing operation part 11 presses the sheet member 43 of the first amplifying liquid holding part 40 via the end part of the assay strip 1. The sheet member 43 breaks by this pressing. The end part of the assay strip 1 enters into the first amplifying liquid holding part 40 from this broken part and is immersed in the first amplifying liquid 41. As described below, the assay strip 1 is formed of a porous material. As a result, in the assay strip 1, the first amplifying liquid 41 is sucked up by the capillary force from the portion of the assay strip 1, which is immersed in the first amplifying liquid 41. Accordingly, the first amplifying liquid 41 is supplied to the assay strip 1. The first amplifying liquid 41 supplied to the assay strip 1 is developed through the inside of the assay strip 1 by capillary action, toward the assay region L1.

In addition, the first pressing operation part 11 is deformed by pressing and then the deformed state is maintained. Accordingly, after the first pressing operation part 11 is pressed, the supply of the first amplifying liquid 41 to the assay strip 1 is continued until all the first amplifying liquid 41 is sucked up.

Similarly, as shown in Fig. 4B, in a case where a pressing force is applied to the second pressing operation part 12, the second pressing operation part 12 is deformed. As shown in Fig. 3, similarly to the first pressing operation part 11, the second pressing operation part 12 of the present example also has a quadrangular pyramid shape, and in a case where a pressing force is applied from above to a region including the apex of the quadrangular pyramid, as shown in Fig. 4B, the second pressing operation part 12 is deformed such that the apex of the quadrangular pyramid sinks into the inside of the case 9. In the second pressing operation part 12 of the present example, an abutting part 12b that abuts on the second amplifying liquid holding part 45 is provided. In a case where the second pressing operation part 12 is deformed, the abutting part 12b presses the second amplifying liquid holding part 45 inside the case 9. The second amplifying liquid holding part 45 is pushed downwardly by pressing, and a sheet member 48, which is described below, of the second amplifying liquid holding part 45 breaks. The second amplifying liquid holding part 45 is arranged at a position facing the surface of the assay strip 1 with an interval from the surface of the assay strip 1. In a case where the second amplifying liquid holding part 45 breaks, the second amplifying liquid 46 held by the second amplifying liquid holding part 45 flows out from the broken part onto the surface of the assay strip 1. Accordingly, the second amplifying liquid 46 is supplied to the assay strip 1.

The second amplifying liquid 46 supplied to the assay strip 1 flows toward the assay region L1 on the surface of the assay strip 1. Then, a part of the second amplifying liquid 46 that has reached the assay region L1 infiltrates the assay region L1. In this manner, similarly to the first amplifying liquid 41, the second amplifying liquid 46 is supplied to the assay strip 1, but unlike the amplifying liquid 41 being developed within the assay strip 1 by the capillary force, the second amplifying liquid 46 flows on the surface of the assay strip 1. Here, the second amplifying liquid 46 is an example of an "amplifying liquid" according to the technology of the present disclosure described in the scope of the claims.

As will be described later, in the assay apparatus 110 of the present example, a plurality of assay flows can be selected. In a case where in the assay apparatus 110, the assay flow for supplying the second amplifying liquid 46 is selected from the assay flows that can be selected in the assay apparatus 110, the second pressing operation part 12 is pressed by the internal mechanism of the assay apparatus 110. Therefore, the second pressing operation part 12 may be depressible by an internal mechanism. On the other hand, in a case of selecting an assay flow in which the cartridge 100 is loaded into the assay apparatus 110 after the supply of the first amplifying liquid 41 and the second amplifying liquid 46, the cartridge 100 is preferably an aspect that the second pressing operation part 12 is also depressible by the user.

(First amplifying liquid holding part)

As shown in Fig. 3, Fig. 4A, and Fig. 4B, the case main body 20 accommodates the assay strip 1 including the carrier 2 along the longitudinal direction. As shown in Fig. 4A, Fig. 4B, and Fig. 5, in the case main body 20, the first amplifying liquid holding part 40 is arranged on one end part (right end part side in Fig. 5) in the longitudinal direction. In the case main body 20, in a portion where the first amplifying liquid holding part 40 is arranged, the first accommodating part 24 having a recess-shaped in accordance with the shape of the first amplifying liquid holding part 40 is formed. One end part of the assay strip 1 is arranged above the first amplifying liquid holding part 40 in a state of being accommodated in the first accommodating part 24.

As shown in Fig. 4A, Fig. 4B, and Fig. 5, the first amplifying liquid holding part 40 holds the first amplifying liquid 41. The first amplifying liquid holding part 40 is constituted of, for example, a container 42 formed of a resin material and having an opening on one surface, and a sheet member 43 that covers the opening of the container 42 and that is breakable. The container 42 is filled with the first amplifying liquid 41, and the opening of the container 42 is sealed by the sheet member 43. The first amplifying liquid holding part 40 is arranged in the first accommodating part 24 in a posture in which the sheet member 43 faces upward.

(Multifunctional member)

In addition, as shown in Fig. 3, Fig. 4A, Fig. 4B, and Fig. 5, the cartridge 100 includes a multifunctional member 30 having a function of accommodating the second amplifying liquid holding part 45. The multifunctional member 30 is arranged on the other end part (end part of the left side on the paper surface in Fig. 5) side of the case main body 20 and above the assay strip 1. The multifunctional member 30 is a member in which the second accommodating part 32 and the flow channel forming part 35 are integrally formed.

The second accommodating part 32 is a part accommodating the second amplifying liquid holding part 45. The second accommodating part 32 has a box shape having an opened upper surface. As shown in Fig. 5, on the bottom of the second accommodating part 32, a protrusion 34 for breaking a sheet member 48, which is described below, of the second amplifying liquid holding part 45, and a supply opening 32A that allows to supply the second amplifying liquid 46 flowed out from the second amplifying liquid holding part 45, toward the carrier 2.

The flow channel forming part 35 is provided to be connected from the second accommodating part 32. The flow channel forming part 35 has a flat plate shape, is arranged at a position facing the assay region L1 or the like in the longitudinal direction of the assay strip 1, and is arranged with an interval from the assay strip 1. Then, between the flow channel forming part 35 and the assay strip 1, a flow channel for flowing the second amplifying liquid 46 flowed out from the second accommodating part 32 toward the assay region L1 or the like is formed. The flow channel forming part 35 is arranged between the observation window 18 and the assay region L1 or the like of the assay strip 1. Therefore, the flow channel forming part 35 is formed of a transparent member and thus the assay region L1 and the like can be observed through the observation window 18.

(Second amplifying liquid holding part)

The second amplifying liquid holding part 45 holds the second amplifying liquid 46. The second amplifying liquid holding part 45 is constituted of, for example, a container 47 formed of a resin material and having an opening on one surface, and a sheet member 48 that covers the opening of the container 47 and that is breakable. The container 47 is filled with the second amplifying liquid 46, and the opening of the container 47 is sealed by the sheet member 48. The second amplifying liquid holding part 45 is arranged in the second accommodating part 32 in a posture in which the sheet member 48 faces downward. Accordingly, the sheet member 48 faces the protrusion 34 in the second accommodating part 32.

The pressing force applied from the second pressing operation part 12 to the second amplifying liquid holding part 45 acts in a direction of pushing down the second amplifying liquid holding part 45 downwardly, whereby the sheet member 43 is pressed against the protrusion 34. The sheet member 48 is pressed against the protrusion 34 to break the sheet member 48. The sheet member 48 is broken, and thus the second amplifying liquid 46 flows out through the flow channel formed by the supply opening 32A at the bottom of the second accommodating part 32 and the flow channel forming part 35.

As shown in Fig. 5, a gap (a clearance) C corresponding to the flow channel for the second amplifying liquid 46 is formed between a back surface 36 of the flow channel forming part 35 of the multifunctional member 30 and the carrier 2 of the assay strip 1. The gap C is, for example, in the range of 0.3 mm to 1 mm. The second amplifying liquid 46 flows out from the supply opening 32A at the bottom of the second accommodating part 32 toward the carrier 2, and the second amplifying liquid 46 that has flowed out flows through the flow channel formed by the gap C and reaches at least above the assay region L1. A part of the second amplifying liquid 46 that has reached the assay region L1 infiltrates into the carrier 2 from the flow channel, and a part thereof flows back and is absorbed in the absorption pad 6 described later.

An absorption pad 6, which is described later, is arranged at one end part of the assay strip 1. In the case main body 20, a support part 22 that supports an end part of the assay strip 1 including the absorption pad 6 is formed at a position facing the absorption pad 6. A second accommodating part 32 of the multifunctional member 30 is arranged above the absorption pad 6. The support part 22 also supports the multifunctional member 30 via the absorption pad 6. In addition, in the case main body 20, a support part 21 that supports a central part of the assay strip 1 is formed.

<Assay strip>

The assay strip 1 includes a carrier 2, a liquid feeding pad 4, and an absorption pad 6. Then, the carrier 2 is fixedly supported on a back pressure-sensitive adhesive sheet 7.

(Carrier)

The carrier 2 is a porous insoluble carrier for developing a sample 50, and includes an assay region L1, a control region L2, and a color development region L3. In addition, the carrier 2 includes a label holding pad 3. The label holding pad 3 constitutes a spotting region on which the sample is spotted from dropping port 16. The color development region L3 is arranged on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

In Fig. 3 and the like, a state in which the assay region L1, the control region L2, and the color development region L3 are expressed as lines is shown, but these are not always expressed. Details is described below, but before developing the sample 50 (see Fig. 6), the first amplifying liquid 41 (see Fig. 5), and the second amplifying liquid 46 (see Fig. 5), the colors of the assay region L1 and the control region L2 are substantially the same as the color of the carrier 2 (for example, white), and thus the assay region L1 and the control region L2 cannot be clearly visually recognized at this stage. In a case where the sample 50 is positive, the color optical density of the assay region L1 is increased by developing the sample 50 and by developing the first amplifying liquid 41 and the second amplifying liquid 46, and thus the assay region L1 is expressed as a line. Since the color development of the assay region L1 is amplified by silver amplification, which is described later, the assay region L1 develops a black color.

The control region L2 is also expressed as a line by the increase of the color optical density in a case where the sample 50, the first amplifying liquid 41, and the second amplifying liquid 46 are developed. Accordingly, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first amplifying liquid 41 is developed. However, the color development region L3 is expressed as an orange color line by changing a dark green color to an orange color in a case where the first amplifying liquid 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

(Carrier - label holding pad)

As shown in Fig. 6, a labeling substance 53 is immobilized to the label holding pad 3. The labeling substance 53 is modified with the first binding substance 52 that specifically binds to the test substance 51 contained in the sample 50. The label holding pad 3 is fixed on the carrier 2 at a position facing the dropping port 16 (see Fig. 3) of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

(Carrier - assay region)

As shown in Fig. 6, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bound to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

(Carrier - control region)

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

(Carrier - color development region)

The color development region L3 contains a substance whose color development state changes by reacting with the first amplifying liquid 41. The color development region L3 shows that the first amplifying liquid 41 has been developed to that region by reacting with the first amplifying liquid 41 to develop a color or change a color. For example, in a case where a mixed aqueous solution of an iron nitrate aqueous solution and citric acid (manufactured by Fujifilm Wako Pure Chemical Corporation, 038-06925) is used as the first amplifying liquid 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first amplifying liquid 41, and the dark green color is changed to an orange color in a case where the first amplifying liquid 41 reaches the color development region L3. The color development region L3 is sometimes referred to as an amplification index region because the timing of supplying the second amplifying liquid 46 after the first amplifying liquid 41 is developed is shown by changing the color development state.

(Binding substance)

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is immobilized in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

(Liquid feeding pad)

The liquid feeding pad 4 is arranged in a state of being contact with one end of the carrier 2 and in the development direction of the sample from the spotting region toward the assay region L1, which is constituted of the label holding pad 3, the first amplifying liquid 41 is fed to the carrier 2 from the upstream side of the spotting region. In the liquid feeding pad 4, in a case where the first pressing operation part 11 is pressed, one end of the liquid feeding pad 4 is immersed in the first amplifying liquid holding part 40. The liquid feeding pad 4 is formed of a porous material and absorbs the first amplifying liquid 41, and the absorbed first amplifying liquid 41 is fed to the carrier 2 by a capillary action.

(Absorption pad)

The absorption pad 6 is arranged in a state of being contact with the other end of the carrier 2 and absorbs the sample 50, the first amplifying liquid 41, and the second amplifying liquid 46, which are developed on the carrier 2. The absorption pad 6 is formed of a porous material and absorbs the sample 50 and the first amplifying liquid 41 to facilitate the development of the sample 50 and the first amplifying liquid 41 in the assay strip 1.

<Amplifying liquid>

In the present embodiment, the amplifying liquid is a two-part amplifying liquid including the first amplifying liquid 41 and the second amplifying liquid 46. The first amplifying liquid 41 is reacted with the second amplifying liquid 46 on the assay strip 1 to amplify the color development in the assay region L1 and the control region L2. In a case where a metal-based labeling substance such as a gold colloid is used as the labeling substance 53 as in the present example, for example, silver amplification is used as a method of amplifying the labeling signal of the labeling substance 53. The first amplifying liquid 41 and the second amplifying liquid 46 are, as an example, amplifying liquids used for silver amplification, and the reaction between the first amplifying liquid 41 and the second amplifying liquid 46 using the labeling substance 53 as a catalyst is an amplification reaction. By the amplification reaction, silver particles 60 having a particle diameter relatively larger than that of the labeling substance 53 are generated.

More specifically, in the present example, the first amplifying liquid 41 is a reducing agent that reduces silver ions, and the second amplifying liquid 46 is a silver ion. In a case where the first amplifying liquid 41, which is a reducing agent, and the second amplifying liquid 46, which is a silver ion, are brought into contact with the labeling substance 53, silver particles are generated, and the generated silver particles deposits on the labeling substance 53 using the labeling substance 53 as a nucleus. By depositing the silver particles on the labeling substance 53, silver particles 60 having a particle diameter larger than that of the labeling substance 53 (see Fig. 6) are generated. Accordingly, the labeling signal issued by the labeling substance 53 is amplified, and as a result, the color development of the labeling substance 53 is amplified in the assay region L1 and the control region L2.

(First amplifying liquid)

As the reducing agent as the first amplifying liquid 41, any inorganic or organic material or a mixture thereof can be used as long as the silver ion used as the second amplifying liquid 46 can be reduced to silver. Preferred examples of the inorganic reducing agent include a reducing metal salt and a reducing metal complex salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by complexing or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of $Fe^{2+}$ is more preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, $\gamma$-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

(Second amplifying liquid)

The solution containing silver ions, which is used as the second amplifying liquid 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

<Immunochromatographic method>

An immunochromatographic method is described with reference to Fig. 6. Here, a case where the sample 50 includes the test substance 51, that is, on the premise that the sample 50 is positive is described.

First, the sample 50 is spotted on the label holding pad 3 which is the spotting region (Step S1). The test substance 51 in the sample 50 spotted on the label holding pad 3, specifically binds to the first binding substance 52 that modifies the labeling substance 53 contained in the label holding pad 3. By the capillary action in the carrier 2, in the carrier 2, the sample 50 is developed from the label holding pad 3 toward the assay region L1. A part of the sample 50 is also developed in a direction opposite to the assay region L1. The arrow S indicates a state in which the sample 50 is developed.

Next, the first amplifying liquid 41 is supplied (Step S2). The first amplifying liquid 41 is supplied from the liquid feeding pad 4 side. The first amplifying liquid 41 is supplied to the carrier 2 through the liquid feeding pad 4 and is developed toward the assay region L1.

After that, the process waits until the first amplifying liquid 41 is developed in the assay region L1 (Step S3 and Step S4). "Wait" shown in Fig. 6 means an action of waiting. The first amplifying liquid 41 is gradually developed toward the assay region L1, and the sample 50 to be developed from the label holding pad 3 and the labeling substance 53 modified with the first binding substance 52 are developed toward the assay region L1 to be pushed by the first amplifying liquid 41 (Step S3).

The test substance 51 in the sample 50 that has reached the assay region L1 is captured by the second binding substance 56 in the assay region L1. That is, the labeling substance 53 bound via the test substance 51 and the first binding substance 52 is captured in the assay region L1. On the other hand, the labeling substance 53 that is not bound to the test substance 51 passes through the assay region L1 without being captured and is captured by the third binding substance 58 in the control region L2.

In a case where the development of the first amplifying liquid 41 proceeds and the first amplifying liquid 41 reaches the color development region L3 (Step S4), the color development region L3 reacts with the first amplifying liquid 41 to change the color development state. In the present example, the color development region L3 is dark green color before reacting with the first amplifying liquid 41, and the dark green color is changed to an orange color by reacting with the first amplifying liquid 41.

After the first amplifying liquid 41 is sufficiently developed, the second amplifying liquid 46 is supplied to the carrier 2 (Step S5). The second amplifying liquid 46 is supplied to the carrier 2 from the absorption pad 6 side of the color development region L3 and is developed toward the assay region L1. Here, the first amplifying liquid 41 contains a reducing agent that reduces silver ions, and the second amplifying liquid 46 contains silver ions. By reacting such the first amplifying liquid 41 with the second amplifying liquid 46, the silver particles 60 are generated using the gold colloidal particles that are the labeling substance 53 as a catalyst. Accordingly, the labeling signal is amplified (Step S6).

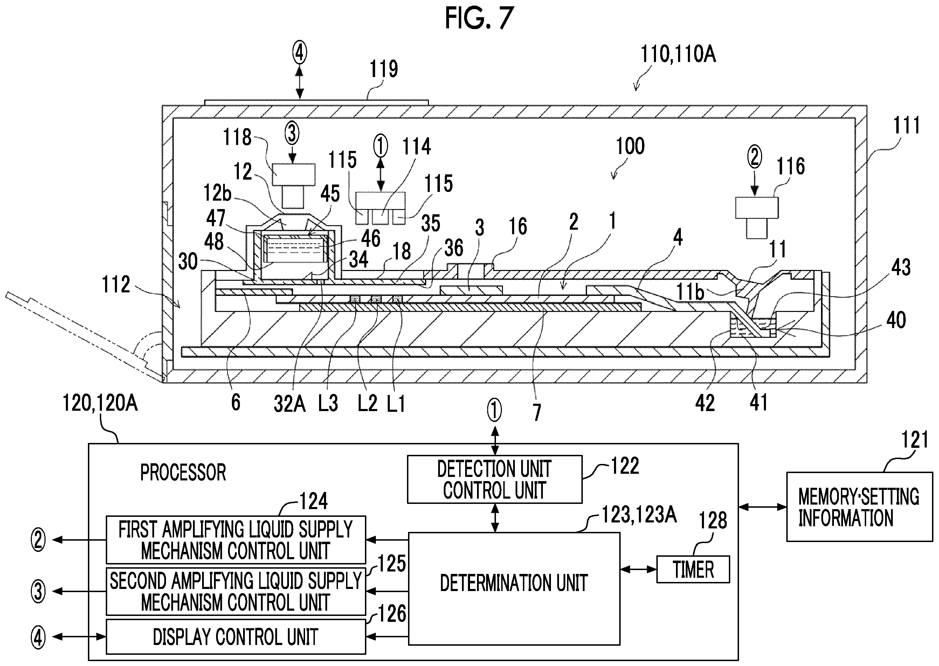

Fig. 7 is a partially broken side view of an assay apparatus 110 in a state where the cartridge 100 is loaded. Hereinafter, a configuration and a function of the assay apparatus 110 is described with reference to Fig. 7.

In the assay apparatus 110 of the present example, for example, the following three assay flows of a first assay flow, a second assay flow, and a third assay flow can be selected. In any of the first to third assay flows, it is necessary that the sample 50 is spotted on the carrier 2 of the cartridge 100 before loading.

The first assay flow is a flow in which an assay is performed on the cartridge 100 in a state where the spotting of the sample 50 and the supply of the first amplifying liquid 41 are started before loading. In the case of the first assay flow, after the cartridge is loaded into the assay apparatus 110, only the supply of the second amplifying liquid 46 to the carrier 2, among the first amplifying liquid 41 and the second amplifying liquid 46, is performed by the assay apparatus 110.

The second assay flow is a flow in which an assay is performed on the cartridge 100 in which only the spotting of the sample 50 is performed before loading. In the case of the second assay flow, after the cartridge is loaded into the assay apparatus 110, the supply of both the first amplifying liquid 41 and the second amplifying liquid 46 to the carrier 2 is performed by the assay apparatus 110.

The third assay flow is a flow in which an assay is performed on the cartridge 100 in a state where the spotting of the sample 50, the supply of the first amplifying liquid 41, and the supply of the second amplifying liquid 46 are started before loading. In the case of the third assay flow, after the cartridge is loaded into the assay apparatus 110, the assay apparatus 110 does not carry out the operation of starting the first amplifying liquid 41 and the second amplifying liquid 46.

Hereinafter, a configuration of the assay apparatus 110 is described in a configuration corresponding to all of the first assay flow, the second assay flow, and the third assay flow, but the first assay flow is premised in the description of the operation of the assay apparatus 110.

(Configuration of assay apparatus 110)

As shown in Fig. 7, the assay apparatus 110 includes a first amplifying liquid supply mechanism 116 and a second amplifying liquid supply mechanism 118 as internal mechanisms. The first amplifying liquid supply mechanism 116 is a mechanism for starting the supply of the first amplifying liquid 41 from the first amplifying liquid holding part 40 to the carrier 2. As the first amplifying liquid supply mechanism 116, for example, an actuator such as a solenoid provided with an electromagnet and a plunger movable with respect to the electromagnet is used. For example, as the plunger moves, the plunger abuts on the first pressing operation part 11 and presses the first pressing operation part 11. The first amplifying liquid supply mechanism 116 is arranged at a position facing the first pressing operation part 11 of the loaded cartridge 100.

The first amplifying liquid supply mechanism 116 is a pressing mechanism that applies a pressing force to the first pressing operation part 11 from the outside by pressing the first pressing operation part 11 of the cartridge 100. In a case where a pressing force is applied to the first pressing operation part 11 by the first amplifying liquid supply mechanism 116, the first amplifying liquid 41 is supplied from the first amplifying liquid holding part 40 to the carrier 2 by the above-described action.

The second amplifying liquid supply mechanism 118 is a mechanism for starting the supply of the second amplifying liquid 46 from the second amplifying liquid holding part 45 to the carrier 2. Also as the second amplifying liquid supply mechanism 118, an actuator such as a solenoid is used similarity to the first amplifying liquid supply mechanism 116. The second amplifying liquid supply mechanism 118 is arranged at a position facing the second pressing operation part 12 of the loaded cartridge 100. The second amplifying liquid supply mechanism 118 is a pressing mechanism that applies a pressing force to the second pressing operation part 12 from the outside by pressing the second pressing operation part 12 of the cartridge 100. In a case where a pressing force is applied to the second pressing operation part 12 by the second amplifying liquid supply mechanism 118, the second amplifying liquid 46 is supplied from the second amplifying liquid holding part 45 to the carrier 2 by the above-described action.

In addition to the cartridge loading part 112, the first amplifying liquid supply mechanism 116, and the second amplifying liquid supply mechanism 118, the assay apparatus 110 further includes a detection unit 114, a processor 120, and a memory 121 in the housing 111. In Fig. 7, the processor 120 and the memory 121 are illustrated outside the housing 111 of the assay apparatus 110, but this is a schematic diagram, and the processor 120 and the memory 121 are actually arranged inside the housing 111.

The detection unit 114 optically detects the color development state of the assay region L1, the control region L2, and the color development region L3, and outputs a detection signal indicating the color development state to the processor 120. The detection unit 114 is, for example, an image sensor such as a complementary metal oxide semiconductor (CMOS) image sensor and a charge coupled device (CCD) image sensor and images the observation region including the assay region L1, the control region L2, and the color development region L3. Then, the imaged image is output from the detection unit 114 to the processor 120.

In addition, as an example, a light source 115 such as a light emitting diode that illuminates the assay region L1, the control region L2, and the color development region L3 during the imaging is provided on both sides of the detection unit 114.

The processor 120 integrally controls each part of the assay apparatus 110. An example of the processor 120 is a central processing unit (CPU) that performs various kinds of control by executing a program. The CPU functions as a control unit having a detection unit control unit 122, a determination unit 123, a first amplifying liquid supply mechanism control unit 124, a second amplifying liquid supply mechanism control unit 125, a display control unit 126, and a timer 128 by executing a program. The memory 121 is an example of a memory connected to or built in the CPU as the processor 120. For example, a control program is stored in the memory 121. The processor 120 is realized by the CPU executing a control program.

In addition to the control program, the memory 121 stores setting information that is preset in order for the processor 120 to perform various kinds of control. As the setting information, information necessary for various determinations by the determination unit 123 described below is stored. The specific shape of the setting information is described below.

The detection unit control unit 122 controls the imaging timing by the detection unit 114.

The first amplifying liquid supply mechanism control unit 124 operates the first amplifying liquid supply mechanism 116 to control the first pressing operation part 11 to be pressed.

The second amplifying liquid supply mechanism control unit 125 operates the second amplifying liquid supply mechanism 118 based on the change in the color development state of the color development region L3 to control the second pressing operation part 12 to be pressed.

The determination unit 123 executes various kinds of determination processing including color development region determination processing, control region determination processing, saturation point determination processing of a sample region density, and positive-negative determination processing, based on the detection signal output by the detection unit 114. As described above, the detection unit 114 outputs the captured image of the observation region including the assay region L1, the control region L2, and the color development region L3. The determination unit 123 executes each of the above-described determination processing based on the captured image.

The color development region determination processing is a processing of determining, based on the density of the color development region L3 among the images output from the detection unit 114, a change in the color development state of the color development region L3, as an example, whether or not the color has changed from a dark green color, which is the color before the reaction with the first amplifying liquid 41, to an orange color. "Presence" of the change in the color development state means that the first amplifying liquid 41 is developed to the color development region L3.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a density change).

The processor 120 operates the second amplifying liquid supply mechanism 118 via the second amplifying liquid supply mechanism control unit 125 in a case where the determination unit 123 determines that the color development state in the color development region L3 has changed. As an example, the determination of the color development state of the color development region L3 and the supply of the second amplifying liquid 46 are performed as follows. First, in a state where the observation region is illuminated by turning on the light source 115, the processor 120 causes the detection unit 114 to perform imaging by operating the detection unit 114. Then, the processor 120 acquires the captured image from the detection unit 114, and determines the change in the color development state of the color development region L3 from the acquired captured image. Here, in a case where the color development state of the color development region L3 has changed, that is, in a case where the color of the color development region L3 has changed from a dark green color to an orange color, it means that the first amplifying liquid 41 has reached the color development region L3, and the assay region L1 and the control region L2 which are on the upstream side of the color development region L3. Then, the processor 120 operates the second amplifying liquid supply mechanism 118 in a case where it is determined that the color development state in the color development region L3 has changed. The processor 120 presses the second pressing operation part 12 of the cartridge 100 by the second amplifying liquid supply mechanism 118. In a case where the second pressing operation part 12 is pressed, the second pressing operation part 12 is deformed to sink toward the second amplifying liquid holding part 45. By this deformation, the sheet member 48 of the second amplifying liquid holding part 45 is pressed against the protrusion 34 to break, and the second amplifying liquid 46 is flowed out from the supply opening 32A of the second accommodating part 32 and supplied onto the carrier 2.

The control region determination processing is a processing of determining presence or absence of a change in the color development state of the control region L2 based on the density of the control region L2 in the image output from the detection unit 114. In the present example, a line is expressed in the control region L2 by the labeling substance 53 being captured in the control region L2 and being subjected to silver amplification. Therefore, specifically, the processor 120 determines presence or absence of the expression of the line in the control region L2 as the control region determination processing.

The saturation point determination processing of the density of the assay region L1 is processing of determining whether or not the density of the assay region L1 has reached the saturation point. The processor 120 determines whether or not the density of the assay region L1 has reached the saturation point based on the time change of the density of the assay region L1 in the image output from the detection unit 114. The processor 120 starts acquisition of the density of the assay region L1 from a preset time point before the density of the assay region L1 is saturated, and determines whether the saturation point has been reached based on the time change of the density of the assay region L1. In the present example, the density of the assay region L1 is changed as the sample 50, the first amplifying liquid 41, and the second amplifying liquid 46 are developed in the assay region L1. The signal amplification of the captured labeling substance is gradually silver-amplified by developing the second amplifying liquid 46 that is finally developed, among the sample 50, the first amplifying liquid 41, and the second amplifying liquid 46, and thus the density also gradually changes.

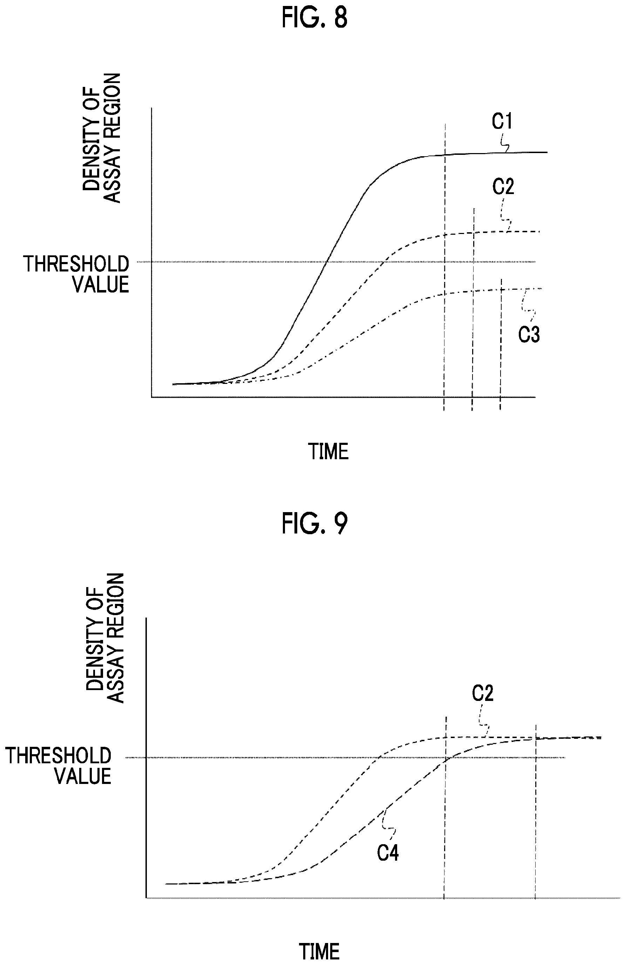

Fig. 8 and Fig. 9 are schematic diagrams showing a general density change of an assay region L1. In Fig. 8 and Fig. 9, the horizontal axis is the time from the start of the supply of the second amplifying liquid 46 and the vertical axis is the density of the assay region L1. Although the final density value differs depending on the content of the test substance in the sample 50, the curves C1, C2, and C3 shown in Fig. 8 are examples of a density change of the assay region L1 in a case where the content of the test substance in the sample 50. As shown in Fig. 8, the density change is drawn as a generally S-shaped curve as shown in the curves C1, C2, and C3 and the density reaches a saturation point. That is, the density of the assay region L1 hardly changes at the beginning of the start of the supply of the second amplifying liquid 46, but starts to gradually increase as time elapses, and then increases rapidly. Furthermore, then, the change in the density of the assay region L1 gradually decreases and finally the density reaches the saturation point. As shown in Fig. 8, the time until the density reaches the threshold value or the time until the density reaches the saturation point varies depending on the content of the test substance included in the sample 50. The curves C1, C2, and C3 in Fig. 8 have a content of the test substance in the sample 50 decreasing in this order. Then, the larger the content of the test substance in the sample 50 is, the shorter a time until the density reaches the threshold value and the saturation point. In Fig. 8, the curve C1 is the shortest. In addition, as shown in the curves C2 and C4 of Fig. 9, even in a case where the contents of the test substance are the same, depending on individual differences in the cartridges 100 and the like, the time until the density reaches the threshold value or the time until the density reaches the saturation point may be different.

In the present embodiment, in the saturation point determination processing, the processor 120 determines that the density of the assay region L1 has reached the saturation point from the time change of the density of the assay region L1. The time change of the density of the assay region L1 is, as an example, a time change of an increase rate, which is an increase amount per unit time in the density of the assay region L1. Then, the processor 120 determines whether the sample is positive or negative by comparing the saturation point with a predetermined density threshold value A for determining whether the sample is positive or negative. Specifically, the processor 120 starts the acquisition of the density of the assay region L1 from the detection unit 114 from a preset time point before the density of the assay region L1 is saturated. The processor 120 performs the saturation point determination of whether or not the density of the assay region L1 has reached a saturation point based on a time change of the density of the assay region L1, and in a case where it is determined in the saturation point determination that the density has reached the saturation point, performs the positive-negative determination of whether the sample is positive or negative.

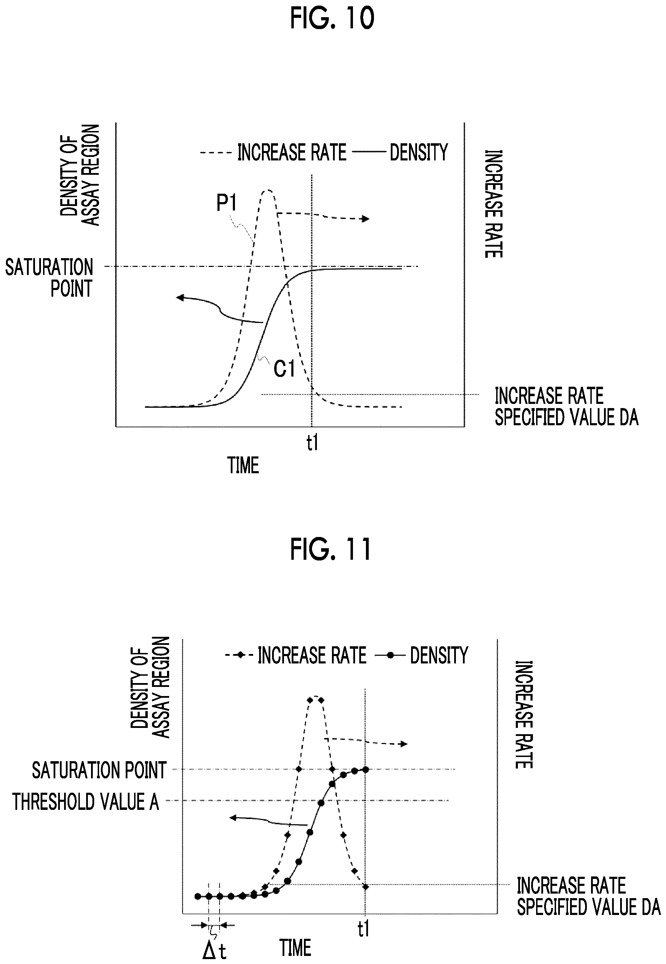

Fig. 10 shows a curve C1 and a curve P1 indicating a change in the slope of the curve C1. That is, the curve P1 represented by a broken line is a time change of the increase rate of the density of the assay region L1. As shown on the curve C1, the density of the assay region L1 shows a S-shaped curve. Therefore, as indicated by the curve P1, the increase rate of the density of the assay region L1 hardly changes at the beginning of the start of the supply of the second amplifying liquid 46, but as the increase rate approaches the saturation point from a certain time point, the increase rate decreases and finally becomes 0. At the time point t1 at which the density of the assay region L1 reaches the saturation point, the increase rate is significantly reduced from the peak value and is a sufficiently small value close to 0. That is, in a case where the increase rate of the density of the assay region L1 exceeds the peak value, then decreases from the peak value, and reaches a predetermined value such as a sufficiently small value close to 0, it can be regarded that the density of the assay region L1 has reached the saturation point. The predetermined value may be 0, or a value within a certain range based on 0 is used. The certain range is, for example, a range of an increase rate of about 10% with respect to the peak value of the curve P1, based on 0 of the curve P1, and is a range that can be regarded that the density has reached the saturation point.

Therefore, as a specific method for the saturation point determination, a method including deriving an increase rate of the density of the assay region L1 indicated by the curve P1 from the time change of the density of the assay region L1 indicated by the curve C1, and in a case where the increase rate of the density of the assay region L1 decreases from the peak value to a predetermined value or less, determining that the density of the assay region L1 has reached the saturation point, can be used. The processor 120 starts the acquisition of the density of the assay region L1 from the detection unit 114 from a preset time point before the density of the assay region L1 is saturated. Since the increase rate changes over time as shown in the curve P1 having one peak value as shown in Fig. 10, in a case where the increase rate decreases, it can be regarded that the increase rate has exceeded the peak value. The preset time point before the density of the assay region L1 is saturated is, for example, a time point after a lapse of a certain period of time from the time when the cartridge 100 is loaded into the assay apparatus 110, a supply start time point at which the supply of the second amplifying liquid 46 is started, a time point after a lapse of a certain period of time from the supply start time point of the second amplifying liquid 46, or the like. In the saturation point determination, any of the time points may be set. In the present example, the supply start time point of the second amplifying liquid 46 is set as the preset time point.

The preset time point, which is a time point at which the acquisition of the density of the assay region L1 is started, may be before the saturation of the density, or may be, for example, after the increase rate exceeds the peak value in the curve P1. Provided that it is more preferable to start the acquisition of the density of the assay region L1 before the peak value is exceeded. This is because, in a case where the acquisition of the density is started before the density exceeds the peak value, it is possible to accurately determine that the density exceeds the peak value and that the density decreases after exceeding the peak value. This is because it is considered that the saturation point can be determined more accurately.

In the density acquisition processing of acquiring the density of the assay region L1, the processor 120 acquires a detection signal of an intensity reflecting the density D(n) of the assay region L1 from the detection unit 114 at certain time interval Δt. Then, the processor 120 derives an increase rate of the density D(n) of the assay region L1 based on the detection signal. The certain time interval Δt is, for example, several seconds to several tens of seconds. In Fig. 11, a curve C1 represented by a black circle marker and a solid line indicates the time change of the density, and a curve P1 represented by a black diamond-shaped marker and a broken line indicates the time change of the increase rate of the density. For example, in a case where the n-th measured density is defined as D(n) and the (n+1)-th measured density is defined as D(n+1), the increase rate ΔD(n) of the density is represented as follows.

$$\Delta D(n) = \{D(n+1) - D(n)\}/\Delta t$$

In the saturation point determination processing, the processor 120 determines whether or not the increase rate ΔD(n) decreases from the peak value to a predetermined value DA (hereinafter, referred to as a specified value DA) or less. Specifically, in a case where the difference DF(n) = ΔD(n) - ΔD(n-1) between the increase rate ΔD(n) and ΔD(n-1) is negative, it can be regarded that the increase rate ΔD(n) decreases. In the saturation point determination processing, in a case where the difference DF(n) is negative, that is, the increase rate ΔD(n) decreases and is not more than the specified value DA, the processor 120 determines that the density of the assay region L1 has reached a saturation point. In a case where the density of the assay region L1 has reached the saturation point, the density D(n) at the time of determining that the density has reached the saturation point is set as the saturation point, and the processor 120 carries out the positive-negative determination processing in the next step.

The positive-negative determination processing is a processing of determining whether the sample 50 is positive or negative based on the density of the assay region L1 acquired from the detection unit 114. In the positive-negative determination processing, the processor 120 compares the density of the saturation point, that is, the density that is acquired after the time point t1 and that is determined to have reached the saturation point in the saturation point determination processing, with a predetermined density threshold value A. Then, in a case where the density D(n) is not less than the density threshold value A, that is, D(n) ≥ A, the processor 120 determines that the sample is "positive", and in a case where D(n) is less than the density threshold value A, the processor 120 determines that the sample is "negative".

In a case where it is determined that the sample is "positive", the processor 120 displays an assay result as "positive" on the monitor 119 via the display control unit 126. In addition, in a case where it is determined that the sample is "negative", the processor 120 displays the assay result as "negative" on the monitor 119 via the display control unit 126.

As an example of the setting information, the memory 121 stores an acquisition start timing of the density of the assay region L1, increase rate specified value DA for determining that the density of the assay region L1 is saturated, a density threshold value A for the positive-negative determination, and the like.

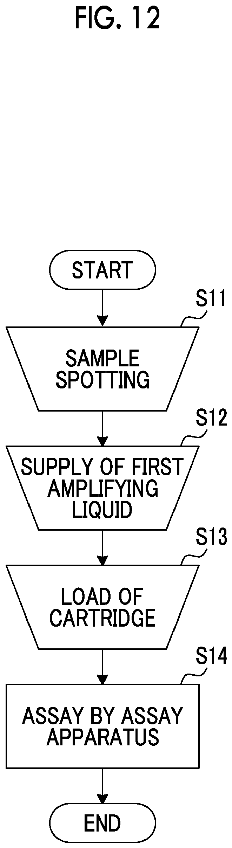

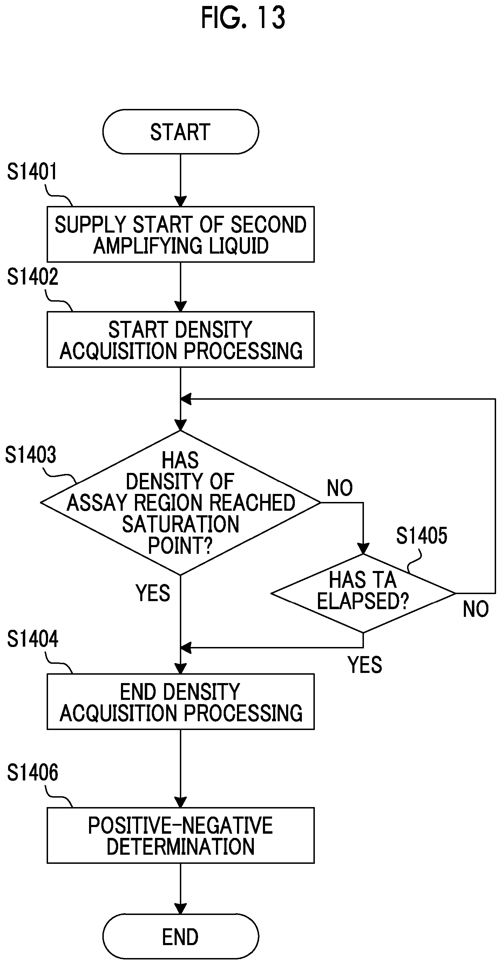

The procedure of the immunochromatographic assay using the assay apparatus 110 according to the present embodiment is described with reference to Fig. 12 and Fig. 13. The procedure of the immunochromatographic assay is described here by using an example of a first assay flow that is an aspect in which the supply of the first amplifying liquid 41 is performed by the user and the supply of the second amplifying liquid 46 is performed by the assay apparatus 110.

(First assay flow)

Fig. 12 is a diagram showing the assay flow.

First, a user adds dropwise the sample 50 from the dropping port 16 of the cartridge 100 onto the spotting region of the carrier 2 (Step S11).

Next, the user presses the first pressing operation part 11 of the cartridge 100 to start the supply of the first amplifying liquid 41 (Step S12).

After that, the user loads the cartridge 100 into the cartridge loading part 112 of the assay apparatus 110 in a state where a power is turned on (Step S13).

An assay of the loaded cartridge 100 is performed in the assay apparatus 110 (Step S14).

From the start of the supply of the first amplifying liquid 41 until the first amplifying liquid 41 is sufficiently developed in the carrier 2, it takes generally about 5 minutes to 10 minutes while varying depending on each cartridge. The timing at which the first pressing operation part 11 is loaded into the cartridge loading part 112 after the user presses the first pressing operation part 11 may be determined according to the convenience of the user within a range of the time required for the development of the first amplifying liquid 41.

Fig. 13 shows a processing procedure of the assay (Step S14) in the assay apparatus 110 shown in Fig. 12. By loading the cartridge 100 into the assay apparatus 110, the assay in the assay apparatus 110 (Step S14 in Fig. 12) is started.

As shown in Fig. 13, in the assay apparatus 110, the processor 120 operates the second amplifying liquid supply mechanism 118 to start the supply of the second amplifying liquid 46 (Step S1401). As described above, the processor 120 starts the supply of the second amplifying liquid 46 in a case where the determination unit 123 determines that the color development state of the color development region L3 has changed.

In the present example, a supply start time point of the second amplifying liquid 46 is set as a preset time point before the density of the assay region L1 is saturated. Therefore, the processor 120 starts the density acquisition processing of acquiring the density of the assay region L1 from the detection unit 114, starting point from the timing of operating the second amplifying liquid supply mechanism 118 (Step S1402).

In the density acquisition processing, the processor 120 repeats the acquisition of the density of the assay region L1 at a certain time interval Δt.

In a case where the density acquisition processing is started, the processor 120 executes the saturation point determination processing of determining whether or not the density of the assay region L1 has reached the saturation point based on the acquired density (Step S1403). In the saturation point determination processing of the present example, as described above, the increase rate of the density is derived, and the increase rate of the density is decreased from the peak value and to be not more than the specified value DA.

In a case where it is determined in the saturation point determination processing of Step S1403 that the density of the assay region L1 has reached the saturation point (Step S1403: YES), the processor 120 terminates the density acquisition processing (Step S1404), and further carries out the positive-negative determination (Step S1406) in the next step. On the other hand, in a case where it is determined that the density of the assay region L1 has not reached the saturation point (Step S1403: No), the processor 120 proceeds to Step S1405.

Step S1405 is a processing for terminating the saturation point determination processing and proceeding to the next step in a case where a preset certain period of time TA has elapsed after the saturation point determination processing is started. For example, in a case where the sample 50 is negative, the density of the assay region L1 does not reach the saturation point. In addition, even in a case where the sample 50 is positive, for example, in a case where the cartridge 100 is loaded into the assay apparatus 110 after the second amplifying liquid 46 is developed because of an operation error of a user, the saturation point determination processing may be performed after the density of the assay region L1 has reached the saturation point. In this case, in a case where the saturation point determination processing is performed based on the increase rate of the density as in the present example, since there is no change in the increase rate, the processor 120 does not determine that the density has reached the saturation point. In such a case, Step S1405 is executed to terminate the saturation point determination processing and proceed to the next step. In Step S1405, the processor 120 repeats the saturation point determination processing based on the newly acquired density until a preset certain period of time TA has elapsed (Step S1405: NO). On the other hand, in Step S1405, in a case where the preset certain period of time TA has elapsed (Step S1405: YES), the processor 120 terminates the saturation point determination processing and proceeds to the next step. The processor 120 terminates the density acquisition processing (Step S1404), and further carries out a positive-negative determination in the next step (Step S1406).

In the positive-negative determination (Step S1406), the density of the saturation point is compared with the density threshold value A (see Fig. 11). In a case where the density $D(n)$ is not less than the density threshold value A, that is, the density $D(n) \geq A$, the processor determines that the sample is "positive", and in a case where the density $D(n)$ is less than the density threshold value A, the processor determines that the sample is "negative". Then, the processor 120 displays the assay result on the monitor 119, and terminates the assay in the assay apparatus 110.

As described above, the processor 120 start the acquisition of the density from the detection unit 114 from the preset time point (in the present example, the supply start time point of the second amplifying liquid 46) before the density of the assay region L1 is saturated, and performs the saturation point determination of determining whether or not the density has reached the saturation point based on the time change of the density. Then, the processor 120 performs a positive-negative determination in a case where it is determined that the density has reached the saturation point in the saturation point determination processing. Therefore, as shown in Fig. 8 and Fig. 9, even in a case where there are individual differences in the density change rate of the assay region L1 for each cartridge 100, the positive-negative determination can be performed in accordance with the density change rate of each cartridge 100. In a case where the concentration of the test substance in the sample is high, the density reaches the saturation point relatively quickly, and thus the positive-negative determination can be performed. Therefore, the assay throughput in a case where a plurality of cartridges 100 are continuously assayed can be improved.

In the present embodiment, as a specific method of determining that the density of the assay region L1 has reached the saturation point, a method including deriving the increase rate of the density from the time change of the density, and in a case where the increase rate of the density decreases from the peak value to a predetermined value or less, determining that the density has reached a saturation point, is used. It can be determined whether or not the density has reached the saturation point by a simple calculation, and the load on the processor 120 is small. However, the method of determining that the density has reached the saturation point is not limited to the above method. For example, only an increase in the density of the curve C1 shown in Fig. 10 may be monitored without deriving the increase rate, and it may be determined that the density has reached the saturation point in a case where the increase in the density is stopped.

In addition, the assay apparatus 110 may be configured to, in a case where the first condition is set to perform the saturation point determination, determine whether or not a predetermined second condition different from the first condition is satisfied, and in a case where the second condition is not satisfied, stop the positive-negative determination, output a determination result with a warning, or perform the positive-negative determination after waiting until the second condition is satisfied. The processor 120 executes a determination of whether or not the second condition is satisfied and a processing in a case where the second condition is not satisfied.

Examples of the second condition include a condition that "a state that affects the determination accuracy of the positive-negative determination has not occurred". Even in a case where the density has reached the saturation point, the determination result of the positive-negative determination is not counted in a case where a state that affects the determination accuracy of the positive-negative determination occurs. In a case where the positive-negative determination is stopped in such a case, unnecessary positive-negative determination is suppressed. In addition, in a case where a determination result with a warning is output instead of stopping the positive-negative determination, it is possible to inform the user that there is a suspicion in the reliability of the determination result. In addition, in a case where there is a possibility that the second condition is satisfied by the lapse of time, an accurate determination result of the positive-negative determination can be obtained by performing the positive-negative determination after waiting until the second condition is satisfied. In this way, by setting the second condition as in the present example separately from the first condition for the saturation point determination, various effects such as suppression of the unnecessary positive-negative determination can be obtained.

Specific examples of the condition that "the state that affects the determination accuracy of the positive-negative determination has not changed" include a condition that "the density of the control region L2 has changed", the condition "the bubbles in the second amplifying liquid in the assay region L1 has disappeared", or the like. In this case, the processor 120 determines whether or not the second condition is satisfied based on the detection signal and the captured image acquired from the detection unit 114.

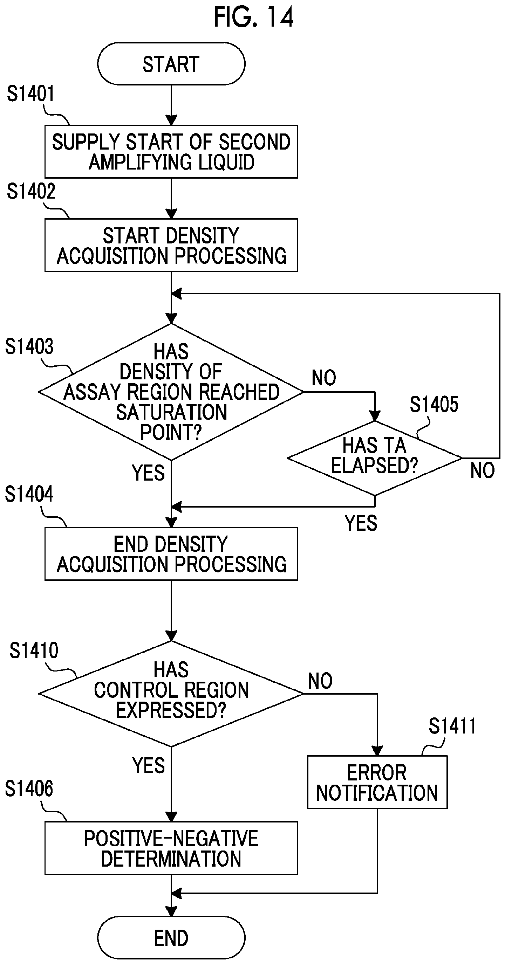

As Modification Example 1 of the processing procedure of the assay in the assay apparatus 110, a case where a step of discriminating presence or absence of a change in the color development state of the control region L2 is included is described with reference to Fig. 14. Fig. 14 shows a processing procedure in a case where a determination step of determining whether or not the control region L2 is expressed is included as a step of discriminating presence or absence of a change in the color development state of the control region L2 in the processing procedure shown in Fig. 13. In Fig. 14, the same steps as those in Fig. 13 are denoted by the same step reference numerals, and detailed description thereof is omitted.

In Modification Example 1 shown in Fig. 14, after it is determined that the density of the assay region L1 has reached the saturation point (Step S1403: YES), and the density acquisition processing is terminated, it is determined whether or not the control region L2 is expressed (Step S1410) before the positive-negative determination (Step S1406). In Step S1410, the processor 120 determines whether or not the control region L2 is expressed from the observation image.

The case where it is determined that the control region L2 is expressed means that the sample 50 has reached the control region L2 and the assay region L1 on the upstream side thereof. In a case where it is determined that the control region L2 is expressed (Step S1410: YES), the positive-negative determination (Step S1406) in the next step is carried out. On the other hand, in a case where it is determined that the control region L2 is not expressed (Step S1410: No), an error is notified (Step S1411) and the processing procedure of the assay terminates. In a case where the control region L2 is not expressed after the development of the second amplifying liquid 46, there is a possibility that the sample 50 has not been spotted.

In addition to stopping the positive-negative determination by notifying an error as described above in a state where the control region L2 is not expressed, a determination result with a warning may be output after performing a positive-negative determination. In addition, the positive-negative determination may be performed after waiting until the color development state of the control region L2 changes. Outputting a determination result with a warning means, for example, adding a warning indicating that the reliability of the determination result is relatively low in a case where the determination result is displayed, or the like.

As described above, in a case where the condition that the control region L2 is expressed is not satisfied, there is a possibility that the sample 50 has not been developed in the assay region L1. In Modification Example 1, in a case where the control region L2 is not expressed, an error is notified and the positive-negative determination is not performed, and thus the inconvenience that the result of the positive-negative determination is displayed even in a case where the sample 50 is not developed in the assay region L1 can be avoided, and the erroneous determination can be suppressed.

Furthermore, as the second condition, a condition that the color development state of the control region L2 "within a preset time" may be included in addition to a condition that "the color development state of the control region L2 changes" In this case, in a case where the second condition of "the color development state of the control region L2 has changed within a preset time" is not satisfied, the processor 120 may notify an error to warn an abnormality, similarly to Modification Example 1.

In a case where the color development state of the control region L2 does not change within the preset time, the abnormality due to the development failure or the like is warned by the error notification, and thus it is possible to take appropriate measures such as re-assay without extending the assay time unnecessarily.

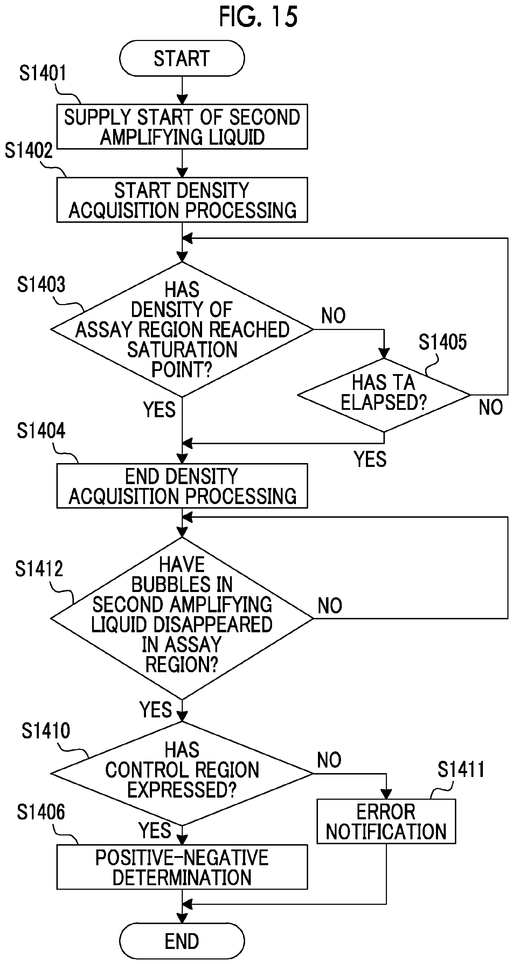

As Modification Example 2 of the processing procedure of the assay in the assay apparatus 110, a case where a step of discriminating presence or absence of bubbles in the amplifying liquid in the assay region L1 is further included is described with reference to Fig. 15. Fig. 15 shows a processing procedure in a case where a determination step of determining whether or not the control region L2 is expressed and a step of discriminating presence or absence of bubbles in the assay region L1 are included in the processing procedure shown in Fig. 13. In Fig. 15, the same steps as those in Fig. 13 and Fig. 14 are denoted by the same step reference numerals, and detailed description thereof is omitted.

In Modification Example 2 shown in Fig. 15, after it is determined that the density of the assay region L1 has reached the saturation point (Step S1403: YES), and the density acquisition processing of the assay region L1 is terminated (Step S1404), it is determined whether or not the bubbles in the second amplifying liquid in the assay region L1 has disappeared (Step S1412) before the positive-negative determination (Step S1406).

In a case where the second amplifying liquid 46 flows out from the supply opening 32A at the bottom of the second accommodating part 32 of the multifunctional member 30 into the gap C serving as a flow channel and in a case where the second amplifying liquid 46 flows through the gap C, bubbles may be generated in the gap C. That is, in a case where the second amplifying liquid 46 is supplied to the carrier 2, bubbles may be generated on the assay region L1 arranged at a position facing the flow channel forming part 35 of the carrier 2 and the periphery of the assay region L1. It is presumed that one of the causes of the generation of bubbles is that the second amplifying liquid 46 flows vigorously into the gap C and the accumulated air and the second amplifying liquid 46 are mixed with each other. In addition, it is also presumed that the cause is that, in a case where the second amplifying liquid 46 that has flowed into the gap C infiltrates into the carrier 2, the air in the carrier 2 floats up on the surface of the carrier 2. It is presumed that bubbles are generated because of at least one of these. The bubbles generated in the second amplifying liquid 46 in the assay region L1 become noise in the saturation point determination and/or the positive-negative determination of the assay region L1. Therefore, in order to perform accurate determination in the assay, it is preferable that the bubbles have disappeared (bubbles are absent) in the second amplifying liquid 46 in the assay region L1.

In the assay region L1, a state is different between a case where bubbles are present in the second amplifying liquid 46 and a case where bubbles are absent, and the difference appears in the image obtained by imaging the observation region. Whether or not the bubbles have disappeared can be determined, for example, by comparing the image without bubbles provided in the memory 121 in advance with the acquired image of the observation region.

Therefore, the processor 120 acquires the captured image from the detection unit 114, compares the acquired captured image with the above-described image without bubbles, and performs the determination that bubbles are present in a case where the difference with the image without bubbles is larger than the certain threshold value, or performs the determination that bubbles are absent in a case where the difference is less than the threshold value.

In a case where it is determined that the bubbles have not disappeared (bubbles are present) (Step S1412: NO), Step S1412 of determining whether or not the bubbles have disappeared is repeated, and the disappearance of bubbles is waited. On the other hand, in a case where it is determined that bubbles are absent (Step S1412: YES), it is further determined whether or not the control region L2 is expressed (Step S1410). The subsequent procedure is as described above in Modification Example 1.

As described above, the bubbles generated in the second amplifying liquid 46 become noise in the saturation point determination and/or the positive-negative determination of the assay region L1. In the present example, in order to perform an accurate determination in the assay, the positive-negative determination is performed after satisfying the condition that "the bubbles in the assay region L1 have disappeared". Therefore, it is possible to suppress the occurrence of an error at the saturation point due to the bubbles in the assay region L1, and it is possible to suppress the erroneous determination.

As in the present embodiment, in a case of including Step S1412 of determining the presence or absence of bubbles and Step S1410 of determining whether or not the control region L2 is expressed, the respective effects of suppressing erroneous determination can be superimposed.

<Second embodiment>

A second embodiment in which the processing in the determination unit 123 of the processor 120 of the above-described assay apparatus 110 is different is described. The second embodiment described here has the same configuration as the configuration of the above-described embodiment described with reference to Fig. 1 to Fig. 7, but the processing in the determination unit 123 of the processor 120 is different from the above-described embodiment. Hereinafter, only differences from the above-described embodiment are described in detail. In order to distinguish from the assay apparatus 110, the processor 120, and the determination unit 123 according to the first embodiment, in the following description of the second embodiment, these are described as the assay apparatus 110A, the processor 120A, and the determination unit 123A.

The determination unit 123A of the processor 120A executes various kinds of determination processing including color development region determination processing, control region determination processing, and positive-negative determination processing, based on the detection signal output by the detection unit 114. The saturation point determination processing of the sample region density, which has been carried out by the determination unit 123 in the first embodiment, is not performed. In addition, the procedure of the positive-negative determination processing is different from that of the first embodiment.

In the positive-negative determination processing of the processor 120A, acquisition of the density from the detection unit 114 from a preset time point before the density of the assay region L1 is saturated is started, and the positive-negative determination is performed based on the density before saturation. Specifically, the processor 120 starts the acquisition of the density of the assay region L1 from the detection unit 114 from a preset time point before the density of the assay region L1 is saturated. The preset time point is, for example, after a certain period of time from the time when the cartridge 100 is loaded into the assay apparatus 110A, a supply start time of the second amplifying liquid 46, after a certain period of time from the supply start time of the second amplifying liquid 46, or the like.

As a specific method for performing positive-negative determination based on the density before saturation, the processor 120A acquires the density of the assay region L1 and compares the acquired density with a predetermined first density threshold value B. Then, in a case where the comparison result satisfies a predetermined first condition, it is determined that the sample 50 is positive.

The predetermined first condition is, for example, a condition that "the density of the assay region L1 exceeds the first density threshold value B". The first condition may be a condition that "in a case where the acquisition of the density of the assay region L1 and the comparison of the acquired density with the first density threshold value B are performed a plurality of times, the number of times that the density of the assay region L1 exceeds the first density threshold value B has reached a predetermined number of times", a condition that "the average value of a plurality of consecutively acquired densities exceeds the first density threshold value B", or the like. In the following, the first condition is described as "the density of the assay region L1 exceeds the first density threshold value B".

The processor 120A repeats the acquisition of the density of the assay region L1 and comparison of the acquired density of the assay region L1 with the first density threshold value B, and determines that the sample 50 is positive at the time point at which the density of the assay region L1 exceeds the first density threshold value B.

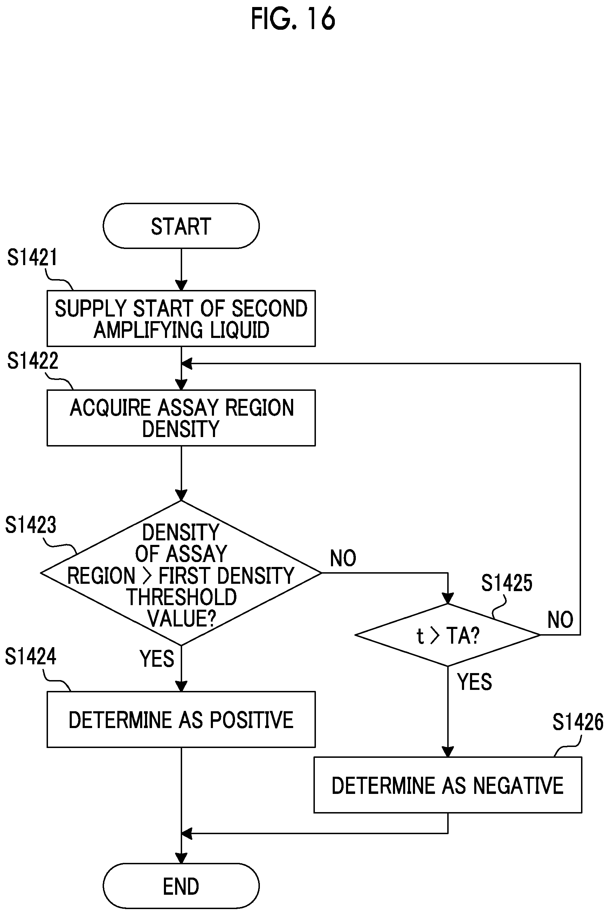

The procedure of the immunochromatographic assay using the assay apparatus 110A according to the second embodiment is performed according to the procedure shown in Fig. 12, but the content of Step S14 of the assay using the assay apparatus 110A is different. Fig. 16 shows a first example of a processing procedure of an assay in the assay apparatus 110A.

As shown in Fig. 16, also in the present example, the processor 120A first operates the second amplifying liquid supply mechanism 118 to start the supply of the second amplifying liquid 46 (Step S1421). This Step S1421 is the same as Step S1401 of Fig. 13, in which the processing procedure of the assay in the assay apparatus 110 according to the first embodiment has been described.

The processor 120A acquires the density of the assay region L1 from the detection unit 114 from the predetermined time point, starting from the timing of operating the second amplifying liquid supply mechanism 118 (Step S1422). Then, the acquired density of the assay region L1 is compared with the first density threshold value B (Step S1423).

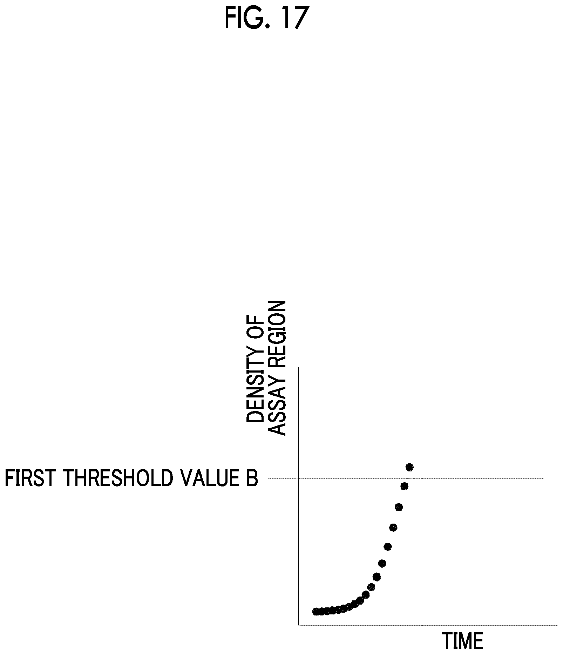

As shown in Fig. 17, in a case where the density of the assay region L1 exceeds the first density threshold value B (Step S1423: YES), the processor 120A determines that the sample is positive (Step S1424).

In a case where the density of the assay region L1 does not exceed the first density threshold value B (Step S1423: NO) and the assay time does not exceed a certain period of time TA, the acquisition of the density of the assay region L1 (Step S1422), and the comparison with the first density threshold value B (Step S1423) are repeated. The assay time exceeds a certain period of time TA (Step S1425: YES), the processor 120A determines that the sample 50 is negative (Step S1426). In the present example, since the density of the assay region L1 is compared with the first density threshold value B, in a case where the density of the assay region L1 has not reached the first density threshold value B even after exceeding a certain period of time TA, it can be determined that the sample is negative. As the certain period of time TA, for example, in a case where the same cartridge 100 is used, the maximum time until the second amplifying liquid 46 is developed and the signal in the assay region L1 is sufficiently amplified is set.

Then, the processor 120A displays the assay result on the monitor 119, and terminates the first example of the processing procedure of the assay in the assay apparatus 110A.

As described with reference to Fig. 8, depending on the content of the test substance in the sample, the time until reaching the threshold value for determining that the sample is positive is different from the time until reaching the saturation point. In addition, as described with reference to Fig. 9, even in a case where the contents of the test substance in the sample are the same, depending on individual differences in the cartridges 100 and the like, the time until reaching the threshold value for determining that the sample is positive may be different from the time until reaching the saturation point. In the processor 120A, acquisition of the density from the detection unit 114 from a preset time point before the density of the assay region L1 is saturated is started, and the positive-negative determination is performed based on the density before saturation. Even in a case where there are individual differences in the density change rate of the assay region L1 for each cartridge, the positive determination can be performed in accordance with the density change rate of each cartridge. Therefore, the throughput of the assay can be improved. In addition, since a positive-negative determination can be performed without waiting for the saturation point of the density, the effect of improving the throughput of the assay is high.

In the present embodiment, the processor 120A starts acquisition of the density of the assay region L1 before the density of the assay region L1 is saturated, and comparison of the acquired density with the first density threshold value B. Then, in a case where the acquired density exceeds the first density threshold value, it is determined that the sample is positive. In a case of being strongly positive where the content of the test substance in the sample 50 is high, the density of the assay region L1 has reached the first density threshold value B before the density of the assay region L1 is saturated, and thus the processor 120A can perform a positive determination without waiting until the saturation point. In this way, in a case of a strongly positive sample, since a positive determination is made without waiting until the saturation point, the improvement of the throughput of the assay can be achieved.

The first condition for determining that the sample is positive is not limited to that "the acquired density exceeds the first density threshold value", and a condition that the sample is regarded to be positive may be used as long as the first condition is satisfied. In a case of being strongly positive, it is considered that the first condition is satisfied at an early stage, and the throughput of the assay for the strongly positive sample can be improved.

As described above, the first condition may be a condition that "in a case where the acquisition of the density of the assay region L1 and the comparison of the acquired density with the first density threshold value B are performed a plurality of times, the number of times that the density of the assay region L1 exceeds the first density threshold value B has reached a predetermined number of times", a condition that "the average value of a plurality of consecutively acquired densities exceeds the first density threshold value B", or the like. Since a positive determination is made based on a plurality of comparisons, it is possible to improve the determination accuracy in a case where the positive determination is performed at a density before saturation.

In the above description, it is described that as a specific method for performing the positive-negative determination based on the density before saturation of the assay region L1, a method in which the processor 120A acquires the density of the assay region L1, compares the acquired density with the predetermined first density threshold value B, and in a case where the comparison result satisfies the predetermined first condition, determines that the sample 50 is positive, is used. However, the method of performing a positive-negative determination based on the density before saturation is not limited to the above. Another method in which the processor 120A performs a positive-negative determination based on the density before saturation is described.

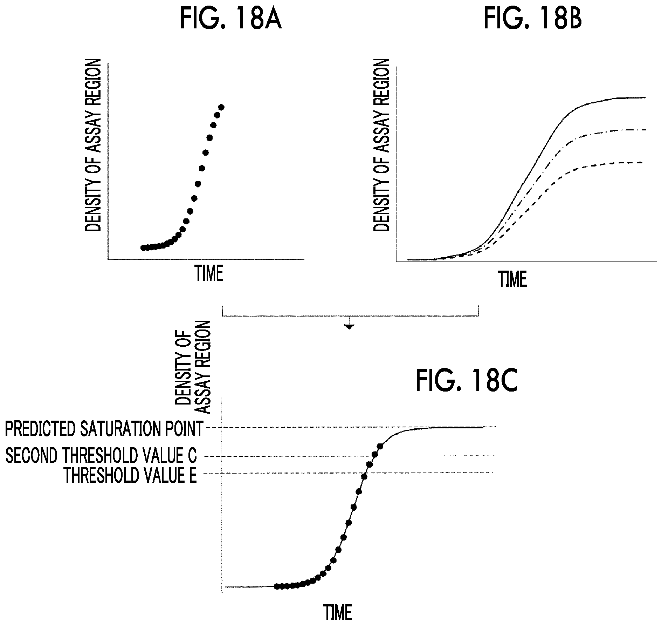

The processor 120A acquires the density of the assay region L1 before saturation from the detection unit 114 a plurality of times. At this time, the processor 120A acquires the density of the assay region L1 before saturation a plurality of times at a certain time interval. The processor 120A acquires the density of the assay region L1 before saturation a plurality of times, and obtains the actually measured data showing the time change of the density before saturation as shown in (A) of Fig. 18. The processor 120A derives a predicted saturation point of the density predicted from the actually measured data based on this actually measured data and the reference data prepared in advance as shown in (B) of Fig. 18 (see (C) of Fig. 18). The reference data is data showing a time change of a reference density for a reference sample including a known amount of the test substance. In the example shown in (B) of Fig. 18, a plurality of reference data for a plurality of reference samples including the test substances having known amounts different from each other are included. Then, the processor 120A derives the predicted saturation point by using one reference data that is most approximated to the actually measured data among the plurality of reference data.

Thereafter, the processor 120A performs a positive-negative determination by comparing the predicted saturation point with a predetermined second density threshold value C (see (C) of Fig. 18). In a case where the predicted saturation point exceeds the second density threshold value C, it is determined that the sample 50 is positive, and in a case where the predicted saturation point is not more than the second density threshold value C, it is determined that the sample 50 is negative.

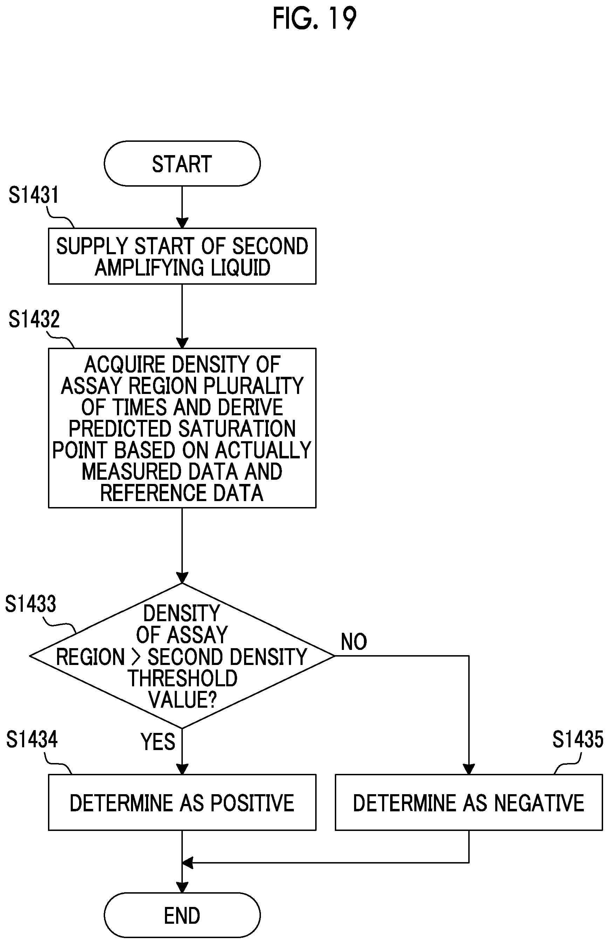

As a second example, Fig. 19 shows a processing procedure of the assay in the assay apparatus 110A in a case where the processor 120A performs the positive-negative determination based on the density before saturation of the assay region L1 as described above.

As shown in Fig. 19, also in the present example, the processor 120A first operates the second amplifying liquid supply mechanism 118 to start the supply of the second amplifying liquid 46 (Step S1431). This Step S1431 is the same as Step S1401 of Fig. 13, in which the processing procedure of the assay in the assay apparatus 110 according to the first embodiment has been described.

The processor 120A acquires the density before saturation of the assay region L1 a plurality of times to obtain actually measured data, and derives a predicted saturation point of the density predicted from the actually measured data based on the actually measured data and the reference data (Step S1432).

Specifically, the processor 120A acquires the density of the assay region L1 at a certain time interval from the detection unit 114 from the predetermined time point before the density of the assay region L1 is saturated, starting from the timing of operating the second amplifying liquid supply mechanism 118, a plurality of times. The preset time point before the density of the assay region L1 is saturated is after a certain period of time from the time when the cartridge 100 is loaded into the assay apparatus 110A, a supply start time of the second amplifying liquid 46, after a certain period of time from the supply start time of the second amplifying liquid 46, or the like, and is a time point before the density is saturated. The certain time interval is, for example, several seconds to several tens of seconds. The processor 120A compares the actually measured data (see (A) of Fig. 18) showing the time change of the density of the assay region L1 acquired at certain time interval with a plurality of reference data prepared in advance (see (B) of Fig. 18), and extracts one reference data most approximated to the actually measured data. Then, the processor 120A fits the extracted reference data to the actually measured data (see (C) of Fig. 18) to derive the predicted saturation point.

The processor 120A compares the derived predicted saturation point with the second density threshold value C (Step S1433).

In a case where the predicted saturation point exceeds the second density threshold value C (Step S1433: YES), the processor 120A determines that the sample is positive (Step S1434).

In a case where the predicted saturation point does not exceed the second density threshold value C (Step S1433: NO), the processor 120A determines that the sample 50 is negative (Step S1435).

Then, the processor 120A displays the assay result on the monitor 119, and terminates the second example of the processing procedure of the assay in the assay apparatus 110A according to the second embodiment.

Similarly to the case of the first example, in the processor 120A, acquisition of the density from the detection unit 114 from a preset time point before the density of the assay region L1 is saturated is started, and the positive-negative determination is performed based on the density before saturation. Accordingly, even in a case where there are individual differences in the density change rate of the assay region L1 for each cartridge, the positive determination can be performed in accordance with the density change rate of each cartridge. Therefore, the throughput of the assay can be improved. In addition, since a positive-negative determination can be performed without waiting for the saturation point of the density, the effect of improving the throughput of the assay is high.

The processor 120A derives a predicted saturation point predicted from the actually measured data based on the actually measured data and the reference data, and performs a positive-negative determination by comparing the predicted saturation point with the second density threshold value C. The processor 120A can perform a positive determination without waiting for the density of the assay region L1 to reach the saturation point. Since the predicted saturation point is derived based on the actually measured data and the reference data, even in a case of being weakly positive as well as strongly positive, a positive determination can be made without waiting until the saturation point, and the effect of improving the throughput of the assay is high.

The second density threshold value C is preferably set to be higher than a threshold value E used in a case of performing the positive-negative determination based on the density of a saturation point of the actually measured data. Here, the threshold value E used in a case of performing the positive-negative determination based on the density of the saturation point of the actually measured data is defined as the average value of the threshold values compared with the saturation point of the density of the assay region L1 in a case where the determination of whether the sample is positive or negative is performed in the assay apparatus in the related art using the cartridge 100.

There is a risk that an error occurs between the predicted saturation point and the actual saturation point. In a case where the predicted saturation point deviates upwardly from the actual saturation point, there is a risk that erroneous determination occurs by using the same value as the threshold value E as the second density threshold value C. By setting the second density threshold value C to be higher than the threshold value E, it is possible to suppress erroneous determination in a case where the predicted saturation point deviates upwardly.

In the above example, a plurality of reference data are provided as the reference data, but only one representative reference data may be provided. In a case of deriving the predicted saturation point based on the actually measured data and one reference data, the predicted saturation point may be derived by performing fitting to the actually measured data by multiplying the reference data by a coefficient or the like. In addition, in a case where a plurality of reference data are provided, an approximated data that approximates to the actually measured data may be obtained by interpolation or extrapolation based on one or two of the reference data that relatively approximates to at least the actually measured data among the reference data to derive the predicted saturation point based on the approximated data. Since the saturation point is predicted by using the approximated data closer to the actually measured data than the reference data provided in advance, the saturation point closer to the actually measured value can be predicted, and the determination accuracy is improved.

The reference data may be a look-up table or a function showing correlation between time and the density. Since a curve along the actually measured data is obtained by the fitting using a function, the saturation point close to the actually measured value can be predicted, and the determination accuracy is high.

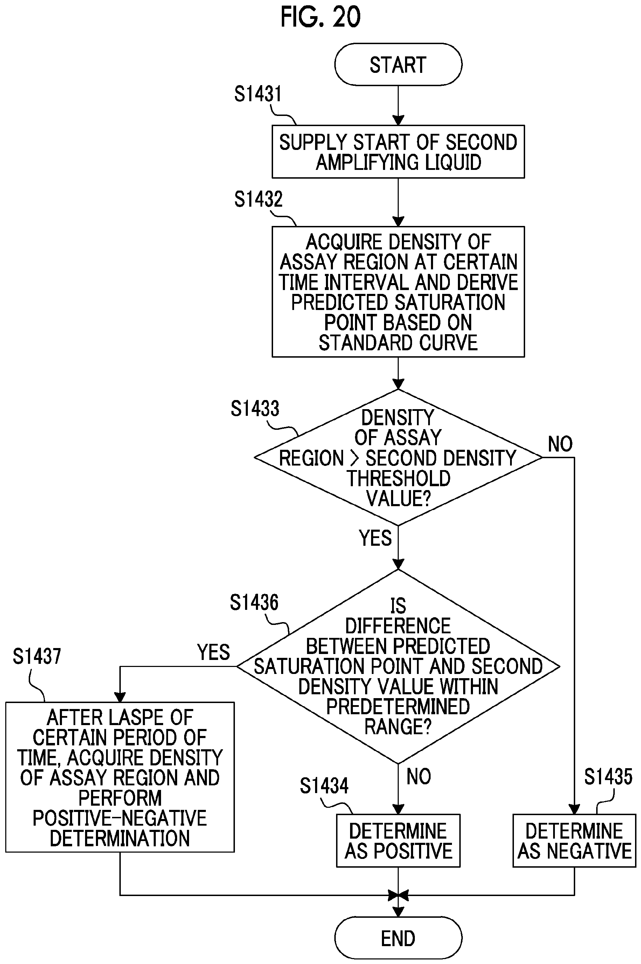

The processor 120A may be configured to, in a case where a difference between the predicted saturation point and the second density threshold value is within a predetermined range, perform the positive-negative determination after waiting until reaching the saturation point without performing the positive-negative determination based on the predicted saturation point. Here, the predetermined range can be appropriately set, but is, for example, a range regarded as a range of an error, and specifically, is a range of $\pm 5\%$ of the second density threshold value, or the like. Fig. 20 shows a modification example of the second example of a processing procedure of an assay in the assay apparatus 110A. In Fig. 20, the same steps as those in Fig. 19 are denoted by the same step reference numerals, and detailed description thereof is omitted.

In the modification example shown in Fig. 20, after the case where the predicted saturation point in Fig. 19 exceeds the second density threshold value C (Step S1433: YES), Step 1436 of determining whether or not the difference between the predicted saturation point and the second density threshold value C is in a predetermined range is included. In a case where the difference between the predicted saturation point and the second density threshold value C is not within the predetermined range (Step S 1436: NO), it is determined that the sample is "positive" as it is (S1434), and the assay is terminated. On the other hand, in a case where the difference between the predicted saturation point and the second density threshold value C is within a predetermined range (Step S1436: YES), the density of the assay region L1 is acquired after a lapse of a certain period of time, and the positive-negative determination is carried out (Step S1437). Here, the certain period of time may be a time sufficient until the density of the assay region L1 reaches the saturation point, may be a certain period of time from the start of the assay, or is a certain period of time from the determination in Step S1436. That is, the density of the assay region L1 acquired after a lapse of a certain period of time is the saturation point, and in Step S1437, the positive-negative determination is performed based on the actual saturation point. Here, in the positive-negative determination, the actual saturation point is compared with the second density threshold value C, and in a case where the saturation point exceeds the second density threshold value C, it is determined that the sample is "positive", or in a case where the saturation point is not more than the density threshold value C, it is determined that the sample is "negative".

There is a risk that an error occurs between the predicted saturation point and the actual saturation point. Therefore, as in the above example, in a case where a difference between the predicted saturation point and the second density threshold value is within a predetermined range, the erroneous determination due to the error of the predicted saturation point can be suppressed by performing the positive-negative determination after waiting until reaching the saturation point without performing the positive-negative determination based on the predicted saturation point.

In a case where the second density threshold value C is set to be higher than the threshold value E used in a case of performing the positive-negative determination based on the density of the saturation point of the actually measured data, as a threshold value to be used for the positive-negative determination in Step S1437, the threshold value E may be used instead of the second density threshold value C.

In the first example of the assay procedure of the assay apparatus 110A according to the second embodiment, the density before the saturation is compared with a predetermined first density threshold value, and in a case where a comparison result satisfies a predetermined first condition, it is determined that the sample is positive. The first example of the assay procedure of the assay apparatus 110A may be configured to, similarly to the case of the assay procedure of the assay apparatus 110 according to the first embodiment, determine whether or not a predetermined second condition different from the first condition is satisfied, and in a case where the second condition is not satisfied, stop the positive-negative determination, output a determination result with a warning, or perform the positive-negative determination after waiting until the second condition is satisfied.

In addition, also in a case where the first condition is set to derive the predicted saturation point of the assay region L1, compare the predicted saturation point with the predetermined second density threshold value, and perform the positive-negative determination, the second example of the assay procedure of the assay apparatus 110A may be configured to determine whether or not a second condition is satisfied, and in a case where the second condition is not satisfied, stop the positive-negative determination, output a determination result with a warning, or perform the positive-negative determination after waiting until the second condition is satisfied.

As described in the assay procedure of the assay apparatus 110A according to the first embodiment, examples of the second condition include a condition that "a state that affects the determination accuracy of the positive-negative determination has not occurred". Specific examples thereof include a condition that "the density of the control region L2 has changed", a condition "the air bubbles are not generated in the amplifying liquid developed in the assay region L1", or the like.

That is, in the first example of the assay procedure shown in Fig. 16 and the second example of the assay procedure shown in Fig. 19, as shown in Modification Example 1 and Modification Example 2 of the processing procedure of the assay in the assay apparatus 110 according to the first embodiment, at least one of the step of determining whether or not the control region L2 is expressed or the step of discriminating the presence or absence of bubbles in the amplifying liquid in the assay region L1 may be included.

An erroneous determination can be suppressed in a case of being configured to determine whether or not a predetermined second condition is satisfied, and in a case where the second condition is not satisfied, stop the positive-negative determination, output a determination result with a warning, or perform the positive-negative determination after waiting until the second condition is satisfied.

In a case where the reference data of the description prepared for determining the density of the assay region L1 is defined as the first reference data, it may be determined in the assay apparatus 110A whether or not the control region L2, which is the second condition, is expressed using, in addition to the first reference data, the second reference data indicating standard time change in a case where the second amplifying liquid 46 is developed in the control region L2.

The time change of the density of the control region L2 draws an S-shaped curve in the same manner as the time change of the density of the assay region L1 shown in Fig. 10 and Fig. 11. The saturation point of the control region L2 does not depend on the content of the test substance in the sample, and is generally constant in a case where the same cartridge 100 is used. Since the standard density change rate of the control region L2 can be known by using the second reference data, it is possible to quickly determine whether or not the second condition based on the density of the control region L2 is satisfied. In addition, since the standard density of the control region L2 can also be grasped by using the second reference data, the determination accuracy of whether or not the control region L2 has reached the target density is also improved.

In each of the above embodiments, although a cartridge using an amplifying liquid has been described as an example of the cartridge 100, the technique of the present disclosure can also be applied to a cartridge in which an amplifying liquid is not used. In this case, the density of the control region L2 and the density of the assay region L1 changes as the labeling substance 53 is captured in each region. As the labeling substance 53 is gradually accumulated, the densities of the assay region L1 and the control region L2 also gradually changes.

In addition, as a cartridge in which an amplifying liquid is not used, a cartridge using a fluorescent label that emits fluorescence by excitation light as a labeling substance, is also conceivable. The technology of the present disclosure can also be applied to a cartridge using such a fluorescent label. For example, in a case where a fluorescent label that emits fluorescence by irradiation with excitation light is used as the label instead of the fine gold particle, a sample is added dropwise to the assay strip 1 and developed toward the assay region L1. In the assay region L1, the amount of fluorescence emission increases in proportion to the amount of the test substance in the sample 50. In the technology of the present disclosure, the density of the assay region L1 is an index that is proportional to the amount of the test substance in the sample 50. In that sense, the amount of the fluorescence emission of the assay region L1 is the same as corresponds to the density of the assay region L1. As a matter of course, the amount of the fluorescence emission also changes like the curve C1 shown in Fig. 10 and the like. Therefore, in the case of a cartridge using a fluorescent label, the amount of the fluorescence emission acquired from the detection unit 114 is acquired as the density of the assay region L1, and the saturation point determination or the like is performed based on the amount of the fluorescence emission.

In the above-described embodiments, as the processor 120, 120A, and a hardware structure of a processing unit as internal configurations thereof that executes various types of processing, such as a detection unit control unit 122, a determination unit 123, 123A, a first amplifying liquid supply mechanism control unit 124, a second amplifying liquid supply mechanism control unit 125, and a display control unit 126, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be configured with one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors.

The disclosure of Japanese Patent Application No. 2021-120870 filed on July 21, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

**Claims**

1.  An immunochromatographic assay apparatus comprising:

    a loading part in which a cartridge including an assay strip having an assay region of which a density varies depending on an amount of a test substance included in a sample is attachably and detachably loaded;
    a detection unit that detects the density of the assay region; and
    a processor configured to perform a positive-negative determination of whether the sample is positive or negative based on the density acquired from the detection unit,
    wherein the processor is configured to

    start acquisition of the density from the detection unit from a preset time point before the density is saturated, and
    perform saturation point determination of whether or not the density has reached a saturation point based on a time change of the density, and in a case where it is determined in the saturation point determination that the density has reached the saturation point, perform the positive-negative determination.

2.  The immunochromatographic assay apparatus according to claim 1,
    wherein the processor is configured to, in the saturation point determination, derive an increase rate of the density from the time change of the density, and in a case where the increase rate of the density decreases from a peak value to a predetermined value or less, determine that the density has reached the saturation point.

3.  An immunochromatographic assay apparatus comprising:

    a loading part in which a cartridge including an assay strip having an assay region of which a density varies depending on an amount of a test substance included in a sample is attachably and detachably loaded;
    a detection unit that detects the density of the assay region; and
    a processor configured to perform a positive-negative determination of whether the sample is positive or negative based on the density acquired from the detection unit,
    wherein the processor is configured to

    start acquisition of the density from the detection unit from a preset time point before the density is saturated, and
    perform the positive-negative determination based on the density before saturation.

4.  The immunochromatographic assay apparatus according to claim 3,
    wherein the processor is configured to, in the positive-negative determination, compare the density before the saturation with a predetermined first density threshold value, and in a case where a comparison result satisfies a predetermined first condition, determine that the sample is positive.

5.  The immunochromatographic assay apparatus according to claim 4,
    wherein the first condition is a case where the density before the saturation exceeds the first density threshold value.

6.  The immunochromatographic assay apparatus according to claim 4,
    wherein in a case where the comparison is performed a plurality of times, the first condition is a case where the number of times that the density before the saturation exceeds the first density threshold value reaches a predetermined number of times, or a case where an average value of the densities before the saturation continuously acquired a plurality of times exceeds the first density threshold value.

7.  The immunochromatographic assay apparatus according to claim 3,
    wherein the processor is configured to, based on actually measured data indicating a time change of the density before the saturation, which is obtained by acquiring from the detection unit a plurality of times, and reference data, which is prepared in advance, indicating a time change of reference density of a reference sample that includes a known amount of the test substance, derive a predicted saturation point of the density, which predicted from the actually measured data, compare the predicted saturation point with a predetermined second density threshold value, and perform the positive-negative determination.

8. The immunochromatographic assay apparatus according to claim 7,
wherein the second density threshold value is set to be higher than a threshold value used in a case of performing the positive-negative determination based on the density of the saturation point of the actually measured data.

9. The immunochromatographic assay apparatus according to claim 7 or 8,

    wherein the reference data includes a plurality of reference data for a plurality of reference samples including the test substances having known amounts different from each other, and
    the processor is configured to derive the predicted saturation point using at least one reference data closest to the actually measured data among the plurality of reference data.

10. The immunochromatographic assay apparatus according to claim 7 or 8,
wherein the processor is configured to obtain approximated data that approximates to the actually measured data by interpolation or extrapolation based on one or two of the reference data that relatively approximates to at least the actually measured data among the reference data, and derive the predicted saturation point based on the approximated data.

11. The immunochromatographic assay apparatus according to any one of claims 7 to 10,
wherein the reference data is a look-up table or a function showing correlation between time and the density.

12. The immunochromatographic assay apparatus according to any one of claims 7 to 11,
wherein the processor is configured to, in a case where a difference between the predicted saturation point and the second density threshold value is within a predetermined range, perform the positive-negative determination after waiting until the density acquired from the detection unit reaches a saturation point without performing the positive-negative determination based on the predicted saturation point.

13. The immunochromatographic assay apparatus according to any one of claims 1 to 12,

wherein the processor is configured to determine whether or not a predetermined second condition is satisfied, and in a case where the second condition is not satisfied, stop the positive-negative determination, output a determination result with a warning, or perform the positive-negative determination after waiting until the second condition is satisfied.

14. The immunochromatographic assay apparatus according to claim 13,
wherein the second condition includes a condition that a state which affects a determination accuracy of the positive-negative determination does not occur.

15. The immunochromatographic assay apparatus according to claim 14,
wherein in a case where the density of the assay region is amplified by developing an amplifying liquid in the assay region, the second condition includes a condition that air bubbles in the amplifying liquid in the assay region have disappeared.

16. The immunochromatographic assay apparatus according to claim 14 or 15,
wherein in a case where the assay strip has a control region in which a color development state changes depending on whether or not at least one of the sample or an amplifying liquid that amplifies the density of the assay region is developed in the assay region, the second condition includes a condition that a color development state of the control region has changed.

17. The immunochromatographic assay apparatus according to claim 16,
wherein in a case where a density of the control region changes by the development of the sample or the amplifying liquid, and reference data, which is prepared for determining the density of the control region, indicating a standard time change of density in a case where the sample or the amplifying liquid is developed is defined as second reference data, the processor is configured to determine whether or not the second condition is satisfied, using the second reference data.

18. The immunochromatographic assay apparatus according to claim 16 or 17,

    wherein the second condition includes a condition that the color development state of the control region has changed within a preset time, and the processor is configured to, in a case where the second condition is not satisfied, stop the positive-negative determination and warn an abnormality.

**19.** The immunochromatographic assay apparatus according to any one of claims 1 to 18
wherein a type that amplifies the density of the assay region by developing an amplifying liquid in the assay region is used as the cartridge.

# FIG. 1

# FIG. 2

# FIG. 3

# FIG. 4A

# FIG. 4B

# FIG. 5

# FIG. 6

| | |
|---|---|
| **S1** SAMPLE SPOTTING | |
| **S2** SUPPLY OF FIRST AMPLIFYING LIQUID | |
| **S3** Wait (DEVELOPMENT OF SAMPLE, LABELING SUBSTANCE, AND FIRST AMPLIFYING LIQUID) | |
| **S4** Wait (DEVELOPMENT OF FIRST AMPLIFYING LIQUID) | |
| **S5** SUPPLY OF SECOND AMPLIFYING LIQUID | |
| **S6** COMPLETION OF SIGNAL AMPLIFICATION | |

## FIG. 7

EP 4 375 647 A1

## FIG. 8

## FIG. 9

## FIG. 10

## FIG. 11

# FIG. 12

START

S11

SAMPLE
SPOTTING

S12

SUPPLY OF FIRST
AMPLIFYING
LIQUID

S13

LOAD OF
CARTRIDGE

S14

ASSAY BY ASSAY
APPARATUS

END

# FIG. 13

START

S1401
SUPPLY START OF SECOND
AMPLIFYING LIQUID

S1402
START DENSITY
ACQUISITION PROCESSING

S1403
HAS
DENSITY OF
ASSAY REGION REACHED
SATURATION
POINT?

NO

S1405
HAS TA
ELAPSED?

NO

YES

YES

S1404
END DENSITY
ACQUISITION PROCESSING

S1406
POSITIVE−NEGATIVE
DETERMINATION

END

# FIG. 14

```
                    ┌─────────────┐
                    │    START    │
                    └──────┬──────┘
                           │
S1401              ┌───────▼────────┐
                   │ SUPPLY START OF SECOND │
                   │  AMPLIFYING LIQUID     │
                   └───────┬────────┘
                           │
S1402              ┌───────▼────────┐
                   │  START DENSITY │
                   │ ACQUISITION PROCESSING │
                   └───────┬────────┘
                           │
                           │◄──────────────────────────┐
S1403          ◄─────────────────►                     │
              ╱   HAS            ╲    NO                │
             ╱  DENSITY OF        ╲──────┐             │
            ╱ ASSAY REGION REACHED ╲     │    S1405    │
            ╲   SATURATION         ╱     ▼             │
             ╲    POINT?          ╱   ◄──────►         │
              ◄─────────────────►    ╱ HAS TA ╲    NO  │
                    │ YES           ╲ ELAPSED? ╱───────┘
S1404               │                ◄──────►
              ┌─────▼──────┐            │ YES
              │ END DENSITY │◄──────────┘
              │ ACQUISITION PROCESSING │
              └─────┬──────┘
                    │
S1410          ◄─────────────────►
              ╱   HAS            ╲    NO
             ╱  CONTROL REGION    ╲────────┐
             ╲   EXPRESSED?       ╱        │   S1411
              ◄─────────────────►          ▼
                    │ YES            ┌──────────────┐
S1406         ┌─────▼──────┐        │    ERROR     │
              │ POSITIVE-NEGATIVE │ │ NOTIFICATION │
              │ DETERMINATION     │ └──────┬───────┘
              └─────┬──────┘               │
                    │◄─────────────────────┘
              ┌─────▼──────┐
              │    END     │
              └────────────┘
```

# FIG. 15

START

S1401 → SUPPLY START OF SECOND AMPLIFYING LIQUID

S1402 → START DENSITY ACQUISITION PROCESSING

S1403 — HAS DENSITY OF ASSAY REGION REACHED SATURATION POINT?

NO → S1405 HAS TA ELAPSED? → NO

YES

S1404 → END DENSITY ACQUISITION PROCESSING

S1412 — HAVE BUBBLES IN SECOND AMPLIFYING LIQUID DISAPPEARED IN ASSAY REGION? → NO

YES

S1410 — HAS CONTROL REGION EXPRESSED? → NO → S1411 ERROR NOTIFICATION

YES

S1406 → POSITIVE–NEGATIVE DETERMINATION

END

# FIG. 16

```
                    START

S1421
          SUPPLY START OF SECOND
            AMPLIFYING LIQUID

S1422
          ACQUIRE ASSAY REGION
                DENSITY

S1423
             DENSITY
            OF ASSAY                  NO
        REGION > FIRST DENSITY  ──────────────┐
            THRESHOLD                          │
              VALUE?                    S1425
                                              NO
                YES                    t > TA?  ──────┐
S1424                                                 │
                                         YES          │
        DETERMINE AS POSITIVE                          │
                                               S1426   │
                               DETERMINE AS NEGATIVE  ─┘

                    END
```

# FIG. 17

# FIG. 18A

DENSITY OF ASSAY REGION

TIME

# FIG. 18B

DENSITY OF ASSAY REGION

TIME

# FIG. 18C

DENSITY OF ASSAY REGION

PREDICTED SATURATION POINT

SECOND THRESHOLD VALUE C

THRESHOLD VALUE E

TIME

# FIG. 19

START

S1431

SUPPLY START OF SECOND
AMPLIFYING LIQUID

S1432

ACQUIRE DENSITY OF
ASSAY REGION PLURALITY
OF TIMES AND DERIVE
PREDICTED SATURATION
POINT BASED ON ACTUALLY
MEASURED DATA AND
REFERENCE DATA

S1433

DENSITY
OF ASSAY
REGION > SECOND DENSITY
THRESHOLD
VALUE?

NO

YES

S1434

DETERMINE AS POSITIVE

S1435

DETERMINE AS NEGATIVE

END

## FIG. 20

START

S1431 — SUPPLY START OF SECOND AMPLIFYING LIQUID

S1432 — ACQUIRE DENSITY OF ASSAY REGION AT CERTAIN TIME INTERVAL AND DERIVE PREDICTED SATURATION POINT BASED ON STANDARD CURVE

S1433 — DENSITY OF ASSAY REGION > SECOND DENSITY THRESHOLD VALUE?

NO

YES

S1436 — IS DIFFERENCE BETWEEN PREDICTED SATURATION POINT AND SECOND DENSITY VALUE WITHIN PREDETERMINED RANGE?

YES

S1437 — AFTER LASPE OF CERTAIN PERIOD OF TIME, ACQUIRE DENSITY OF ASSAY REGION AND PERFORM POSITIVE-NEGATIVE DETERMINATION

NO

S1434 — DETERMINE AS POSITIVE

S1435 — DETERMINE AS NEGATIVE

END

<table>
<tr><td colspan="2"><strong>INTERNATIONAL SEARCH REPORT</strong></td><td>International application No.<br><strong>PCT/JP2022/026644</strong></td></tr>
</table>

**A. CLASSIFICATION OF SUBJECT MATTER**

***G01N 21/78***(2006.01)i; ***G01N 21/64***(2006.01)i; ***G01N 33/543***(2006.01)i
FI: G01N21/78 A; G01N33/543 521; G01N21/64 F

According to International Patent Classification (IPC) or to both national classification and IPC

**B. FIELDS SEARCHED**

Minimum documentation searched (classification system followed by classification symbols)

G01N21/78; G01N21/64; G01N33/543

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2015-068764 A (TOPPAN PRINTING CO., LTD.) 13 April 2015 (2015-04-13) paragraphs [0001]-[0062], fig. 1-2 | 1-6 |
| Y | | 13-19 |
| Y | JP 2011-075366 A (FUJIFILM CORP.) 14 April 2011 (2011-04-14) paragraphs [0068], [0084]-[0098], fig. 5A-6D | 13-19 |
| Y | JP 2011-215023 A (CENTRAL RES. INST. OF ELECTRIC POWER IND.) 27 October 2011 (2011-10-27) paragraph [0112] | 15 |
| Y | JP 2005-529334 A (CHENGDU KUACHANG SCIENCE & TECHNOLOGY CO., LTD.) 29 September 2005 (2005-09-29) paragraph [0038] | 19 |
| A | US 2019/0323961 A1 (ALERE SWITZERLAND GMBH) 24 October 2019 (2019-10-24) | 1-19 |

☐ Further documents are listed in the continuation of Box C.   ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **02 September 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/026644**

| Patent document cited in search report | | | Publication date (day/month/year) | Patent family member(s) | | | Publication date (day/month/year) |
|---|---|---|---|---|---|---|---|
| JP | 2015-068764 | A | 13 April 2015 | (Family: none) | | | |
| JP | 2011-075366 | A | 14 April 2011 | US | 2011/0072885 | A1 | |
| | | | | paragraphs [0100], [0118]-[0132], fig. 5A-6D | | | |
| | | | | EP | 2309256 | A2 | |
| | | | | CN | 102033109 | A | |
| JP | 2011-215023 | A | 27 October 2011 | (Family: none) | | | |
| JP | 2005-529334 | A | 29 September 2005 | US | 2005/0153382 | A1 | |
| | | | | paragraph [0035] | | | |
| | | | | WO | 2003/104808 | A1 | |
| | | | | EP | 1568995 | A1 | |
| | | | | CN | 1402003 | A | |
| | | | | CA | 2488237 | A1 | |
| | | | | CN | 1672051 | A | |
| | | | | KR | 10-2005-0010509 | A | |
| US | 2019/0323961 | A1 | 24 October 2019 | JP | 2004-361409 | A | |
| | | | | JP | 2011-257414 | A | |
| | | | | JP | 2004-361406 | A | |
| | | | | JP | 2004-361410 | A | |
| | | | | JP | 2011-215152 | A | |
| | | | | US | 2018/0188170 | A1 | |
| | | | | US | 2014/0377879 | A1 | |
| | | | | US | 2013/0149776 | A1 | |
| | | | | US | 2011/0178723 | A1 | |
| | | | | US | 2010/0172802 | A1 | |
| | | | | US | 2008/0213875 | A1 | |
| | | | | US | 2005/0036148 | A1 | |
| | | | | US | 2005/0037510 | A1 | |
| | | | | US | 2005/0037511 | A1 | |
| | | | | EP | 1484611 | A2 | |
| | | | | EP | 2293068 | A1 | |
| | | | | EP | 1484601 | A2 | |
| | | | | EP | 2128604 | A1 | |
| | | | | EP | 2385363 | A2 | |
| | | | | EP | 1484613 | A2 | |
| | | | | EP | 2282204 | A1 | |
| | | | | CA | 2468014 | A1 | |
| | | | | CA | 2468188 | A1 | |
| | | | | CN | 1573315 | A | |
| | | | | CN | 101598740 | A | |
| | | | | CN | 1573316 | A | |
| | | | | CN | 101650298 | A | |
| | | | | CN | 102162789 | A | |
| | | | | CN | 102162790 | A | |
| | | | | CA | 2468009 | A1 | |
| | | | | CN | 1573314 | A | |
| | | | | CN | 101639451 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009139254 A
- JP 2012103150 A
- US 6020117 A
- JP 2021120870 A